# EUROPEAN PATENT SPECIFICATION

(11) **EP 1 295 619 B1**
(45) Date of publication and mention of the grant of the patent: **09.11.2011**
(21) Application number: 02020884.9
(22) Date of filing: 18.09.2002
(51) Int. Cl.: A61M 1/02, A61M 1/36, A61M 1/38

(54) **Platelet collection apparatus**
Apparat zur Gewinnung von Blutplättchen
Appareil pour la collecte de plaquettes sanguines

(30) Priority: 19.09.2001 JP 2001285182
(43) Date of publication of application: 26.03.2003
(73) Proprietor: Terumo Kabushiki Kaisha, Tokyo 151-0072 (JP)
(72) Inventor: Suzuki, Atsushi, Shibuya-ku Tokyo 151-0072 (JP)
(74) Representative: TBK

(56) References cited:
- US-A- 5 637 082

## Description

### BACKGROUND OF THE INVENTION

The present invention relates to a platelet collection apparatus for separating blood into predetermined blood components.

To effectively utilize collected blood and reduce donor's burden, the collected blood is separated into various components centrifugally and components required for a donee are collected, whereas remaining components are returned to the donor.

In obtaining a platelet product in this platelet collection process, the blood donated from the donor is introduced to a platelet collection circuit to separate it into four components of plasma, white cells, platelets, and red cells by a centrifugal separator called a centrifugal bowl installed in the platelet collection circuit. Then, the platelets are collected in a container to produce a platelet product, the plasma is collected in a separate container to produce a plasma product or the material for a plasma fraction product, and the remaining plasma, the white cells, and the red cells are returned to the donor.

As methods of collecting the platelets, the method disclosed in U.S.P. 5,637,082 is described below. In the method, blood is preliminarily collected from a donor before platelets are collected to obtain blood information (hematcrit value, platelet concentration) from the blood preliminarily collected. Data of the blood information and a target number of to-be-collected platelets are inputted to the platelet collection apparatus to thereby compute a necessary number of platelet collection operation cycles. A platelet collection operation is performed in a computed number of cycles.

However, an analyzing apparatus for providing blood information from the blood preliminarily collected may make an error in measurement or an operator may make an error in inputting information to the platelet collection apparatus. In this case, based on erroneous data of blood information, the number of platelet collection operation cycles is computed. The platelet collection operation is performed in the computed number of cycles. Therefore the operator think that a target number of platelets is obtained. However, an examination made after collection of platelets reveals that platelets have been collected more than a predetermined number of platelets. When an excessive amount of platelets is collected, the amount of platelets is adjusted before a blood transfusion is performed and an extra amount thereof is discarded to prevent an excessive amount of platelets from being administered to a patient.

Accordingly, it is an object of the present invention to provide a platelet collection apparatus capable of preventing collection of an excessive amount of platelets.

Depending on a blood condition of a donor, there is a case in which platelets can be collected in an amount more than expected. In this case, it is possible to alter a target number of units of to-be-collected platelets to a number larger than an initial target value. However, it is impossible to understand such a situation in a platelet collection operation. Thus an excessive amount of platelets is collected, as described above and an extra amount thereof is discarded.

### SUMMARY OF THE INVENTION

The object described above is attained by a platelet collection apparatus separating blood into a plurality of components by means of a centrifugal separator having a blood-storage space therein and collecting at least platelets of said components which comprises a platelet collection circuit having a first line connecting an inlet of said centrifugal separator and a blood collection means with each other, a second line connected with an outlet of said centrifugal separator, and a platelet collection bag connected with said second line; a turbidity sensor disposed on said second line; and a function of computing a number of platelet collection cycles and an expected number of to-be-collected platelets or a value related thereto from data of a donor's hematcrit value, platelet concentration data, and a target number of said to-be-collected platelets, all of which are inputted to said platelet collection apparatus before a platelet collection operation is performed, a function of determining whether there is a tendency to increase or decrease in a number of collected platelets by using an estimated number of to-be-collected platelets computed by using an amount of plasma containing high-concentration platelets and a platelet concentration obtained from a turbidity sensor when said plasma containing high-concentration platelets is collected or data related to said platelet concentration and by using said expected number of to-be-collected platelets or said value related thereto; and a function of decreasing an amount of platelets to be collected in a subsequent platelet collection operation if said function of determining on an increase/decrease tendency in the number of collected platelets determines that there is a tendency to increase in the number of said collected platelets.

Further, the object described above is attained by a platelet collection apparatus separating blood into a plurality of components by means of a centrifugal separator having a blood-storage space therein and collecting at least platelets of said components which comprises a platelet collection circuit having a first line connecting an inlet of said centrifugal separator and a blood collection means with each other, a second line connected with an outlet of said centrifugal separator, and a pletelet collection bag connected with said second line: a turbidity sensor disposed on said second line; a platelet-concentration peak situation determination function of detecting a peak of a platelet concentration by using signals detected by said turbidity sensor, when high-concentration platelets-containing plasma is collected in a platelet collection operation and determining whether said detected peak of said platelet concentration is on a normal level or on an excessive level; and a function of decreasing a number of platelets to be collected in said platelet collection operation, when said platelet-concentration peak situation determination function determines that said peak of said platelet concentration is on said excessive level.

### BRIEF DESCRIPTION OF THE DRAWINGS

Fig. 1 is a plan view showing an example of the construction of a platelet collection circuit used in a platelet collection apparatus of the present invention.

Fig. 2 is a partially cutaway sectional view showing a state in which a drive unit is mounted on a centrifugal separator used in the platelet collection circuit.

Fig. 3 is a concept view showing a platelet collection apparatus, according to an embodiment of the present invention, having the platelet collection circuit mounted thereon.

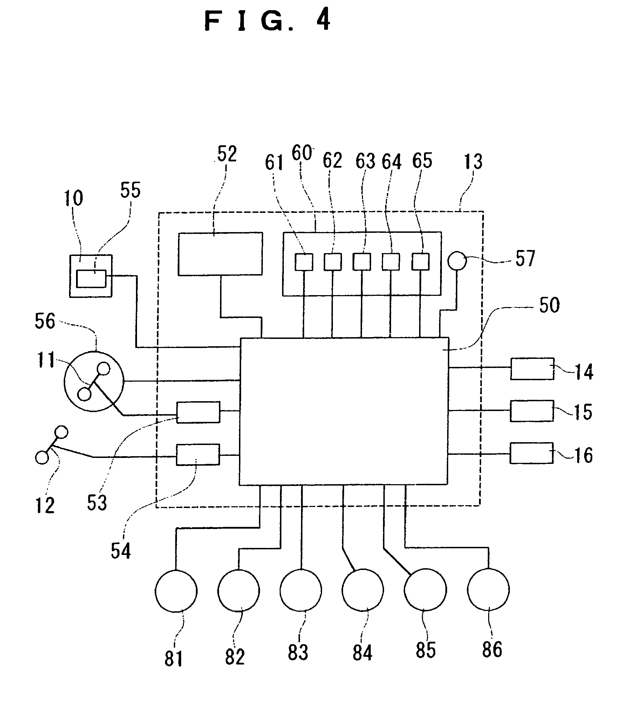

Fig. 4 is a block diagram showing a controller used in the platelet collection apparatus of the present invention.

Fig. 5 is a flowchart for describing the operation of the platelet collection apparatus according to an embodiment of the present invention in the range from a start until an acceleration plasma circulation step.

Fig. 6 is a flowchart for describing the operation of the platelet collection apparatus according to an embodiment of the present invention in the range from a small-amount plasma collection step until a computation of a ROM value for computing an estimated number of to-be-collected platelets.

Fig. 7 is a flowchart for describing the operation of the platelet collection apparatus according to an embodiment of the present invention in the range from the computation of the estimated number of to-be-collected platelets until a blood return step.

Fig. 8 is a flowchart for describing an operation of a mode, for allowing alteration of number of units of to-be-collected platelets, to be executed in the platelet collection apparatus according to an embodiment of the present invention.

Fig. 9 is a flowchart for describing the operation of the platelet collection apparatus according to an embodiment of the present invention in the range from a buffy coat return step until the acceleration plasma circulation step.

Fig. 10 is a flowchart for describing the operation of the platelet collection apparatus according to an embodiment of the present invention in the range from small-amount plasma collection step until the computation of the ROM value for computing the estimated number of to-be-collected platelets.

Fig. 11 is a flowchart for describing the operation of the platelet collection apparatus according to an embodiment of the present invention in the range from the computation of the estimated number of to-be-collected platelets until the blood return step.

Fig. 12 is a flowchart for describing the operation of the platelet collection apparatus according to an embodiment of the present invention in the range from the small-amount plasma collection step until a platelet collection step.

Fig. 13 is a flowchart for describing the operation of the platelet collection apparatus according to an embodiment of the present invention in the range from the ROM value for computing the estimated number of to-be-collected platelets until the blood return step.

Fig. 14 is a flowchart for describing the operation of the platelet collection apparatus according to another embodiment of the present invention in the range from a start until an acceleration plasma circulation step.

Fig. 15 is a flowchart for describing the operation of the platelet collection apparatus according an embodiment of the present invention in the range from the small-amount plasma collection step until the platelet collection step.

Fig. 16 is a flowchart for describing the operation of the platelet collection apparatus according an embodiment of the present invention in the range from the ROM value for computing the estimated number of to-be-collected platelets until the blood return step.

Fig. 17 is a flowchart for describing the operation of the platelet collection apparatus according an embodiment of the present invention in the range from the buffy coat return step until the acceleration plasma circulation step.

### DETAILED DESCRIPTION OF THE PREFERRED EMBODIMENTS

A platelet collection apparatus according to the present invention will be described with reference to embodiments shown in the accompanying drawings.

A platelet collection apparatus 1 according to the present invention has a platelet collection circuit 2 including a centrifugal separator 20 having a rotor 142 having an internal blood storage space and an inlet 143 and an outlet 144 both communicating with the blood internal storage space and centrifugally separating blood introduced into the internal blood storage space through the inlet 143 by a rotation of the rotor 142 into components; a first line 21 for connecting a Hood collection needle 29 or a connector (not shown) of a blood collection instrument and the inlet 143 of the centrifugal separator 20 with each other; a second line 22 connected to the outlet 144 of the centrifugal separator 20; a third line 23, connected to the first line 21, for injecting an anticoagulant to the blood; a plasma collection bag 25 having a first tube 25a connected with the first line 21 and a second tube 25b connected with the second line 22; and a platelet collection bag 26 connected with the second line 22.

The platelet collection apparatus 1 of the present invention further includes a liquid supply pump 11 (first liquid supply pump) mounted on the first line 21. The platelet collection apparatus 1 has a function of computing the number of platelet collection cycles and the expected number of to-be-collected platelets or a value related thereto from the data of a donor's hematcrit value, platelet concentration data, and the target number of to-be-collected platelets or a value related thereto, all of which are inputted to the platelet collection apparatus 1 before a platelet collection operation is performed. The data of a donor's hematcrit value, platelet concentration data and the target number of to-be-collected platelets or a value related thereto are inputted to the platelet collection apparatus 1 before a platelet collection operation is performed.

The platelet collection apparatus of the present invention has a function (hereinafter referred to as function of determining on an increase/decrease tendency in the number of collected platelets) of determining whether there is a tendency to increase or decrease in the number of collected platelets after plasma (PC) containing high-concentration platelets is collected in a plasma collection operation. The function of determining on an increase/decrease tendency determines by using data, related to the concentration of platelets, obtained from a turbidity sensor when the plasma containing high-concentration platelets is collected, the expected number of to-be-collected platelets or a value related thereto And, the platelet collection apparatus 1 further includes a function of decreasing the amount of platelets to be collected in a subsequent platelet collection operation in the case where the function of determining on an increase/decrease tendency in the number of collected platelets has determined that there is a tendency to increase in the number of collected platelets.

Also, The platelet collection apparatus of the present invention has a function (hereinafter referred to as function of determining on an increase/decrease tendency in the number of collected platelets) of determining whether there is a tendency to increase or decrease in the number of collected platelets after plasma (PC) containing high-concentration platelets is collected in a plasma collection operation. The function of determining on an increase/decrease tendency determines by using data, related to the concentration of platelets, obtained from a turbidity sensor when the plasma containing high-concentration platelets is collected, the expected number of to-be-collected platelets or a value related thereto. And, the platelet collection apparatus further includes a function of altering an inputted target number of units of to-be-collected platelets in the case where the function of determining on an increase/decrease tendency in the number of collected platelets has determined that the number of units of collected platelets is on "unit-increased level". The platelet collection apparatus further includes a function of increasing the amount of platelets to be collected in a subsequent platelet collection operation in the case where the function of altering the target number of units of to-be-collected platelets has altered the target number of units of to-be-collected platelets.

It is preferable that the platelet collection apparatus of the present invention has the function of determining whether there is a tendency to increase or decrease in the number of collected platelets by using data, related to the concentration of platelets, obtained from the turbidity sensor, the expected number of to-be-collected platelets or a value related thereto and determining on a "level of tendency to increase" and a "unit-increased level" higher than the "level of tendency to increase" after the plasma (PC) containing high-concentration platelets is collected in the plasma collection operation. The platelet collection apparatus further includes the function of altering an inputted target number of units of to-be-collected platelets in the case where the function of determining on an increase/decrease tendency in the number of collected platelets has determined that the number of units of platelets is on the "unit-increased level". The platelet collection apparatus further includes the function of increasing the amount of platelets to be collected in a subsequent platelet collection operation in the case where the function of altering the target number of units of to-be-collected platelets has altered the target number of units of to-be-collected platelets. The platelet collection apparatus further includes the function of decreasing the amount of platelets to be collected in a subsequent platelet collection operation in the case where the function of altering the target number of units of to-be-collected platelets has not altered the target number of units of platelets in a predetermined period of time after termination of the operation of the function of altering the target number of units of to-be-collected platelets and in the case where the function of determining on an increase/decrease tendency in the number of collected platelets has determined that the number of collected platelets is on the "level of tendency to increase".

The platelet collection apparatus of the present invention has a platelet-concentration peak situation determination function of detecting a peak of the platelet concentration by using signals detected by the turbidity sensor when the high-concentration platelets-containing plasma is collected in the platelet collection operation and determining whether the detected peak of the platelet concentration is on a "normal level" or an "excessive level". The platelet collection apparatus further includes a function of decreasing the number of platelets to be collected in the platelet collection operation, when the platelet-concentration peak situation determination function has determined that the peak of the platelet concentration is on the "excessive level".

The platelet collection apparatus 1 has a centrifugal separator drive unit 10 for rotating the rotor 142 of the centrifugal separator 20; the first liquid supply pump 11 to be used for the first line 21; a second liquid supply pump 12 to be used for the third line 23; a plurality of flow path shutter means 81, 82, 83, 84, 85, and 86 for opening/closing flow paths of the platelet collection circuit 2, and a controller 13 for controlling the centrifugal separator drive unit 10, the first liquid supply pump 11, the second liquid supply pump 12, and a plurality of the flow path shutter means 81, 82, 83, 84, 85, and 86.

The platelet collection circuit 2 will be described in detail below.

The platelet collection circuit 2 is provided to collect platelets.

The platelet collection circuit 2 includes a blood collector such as a blood collection needle 29 or a connector (connector of blood collector) connected with the blood collection needle 29 or with a blood collector having a blood pool connector; the first line 21 (blood collection/blood return line) connecting the blood collection needle 29 or the connector of the blood collector and the inlet 143 of the centrifugal separator 20 with each other and having a first pump tube 21g; the second line 22 for connecting the outlet 144 of the centrifugal separator 20 and the first line 21 with each other; the third line (anticoagulant injection line) 23 connected with a position, of the first line 21, near the blood collection needle 29 and having a second pump tube 23a; a plasma collection bag 25 having the first tube 25a connected with a branch connector 21f positioned nearer to the collection needle 29 than a pump tube 21g of the first line 21 and the second tube 25b connected with the second line 22; the platelet collection bag 26 having a third tube 26a connected with the second line 22; and a buffy coat collection bag 27 having a fourth tube 27a connected with the second line 22. Instead of the blood collection needle 29, the platelet collection circuit 2 may employ a connector (such as a metal or plastic needle) to be connected with a blood pool such as a blood bag.

A known metal needle is used as the blood collection needle 29. The first line 21 includes a blood collection needle-side part 21a with which the blood collection needle 29 is connected and a centrifugal separator-side part 21b with which the inlet 143 of the centrifugal separator 20 is connected. The blood collection needle-side part 21a is formed of a plurality of soft plastic tubes. The blood collection needle-side part 21a has the following elements arranged as viewed from the side of the blood collection needle 29: a branching connector 21c for connecting the blood collection needle-side part 21a and the third line 23 with each other; a chamber 21d for removing bubbles and micro-aggregates; a branching connector 21e for connecting the blood collection needle-side part 21a and the second line 22 with each other; and a branching connector 21f for connecting the first line 21 and the first tube 25a of the plasma collection bag 25 with each other. The chamber 21d is connected to an air-permeable and germ-blocking filter 21i. The centrifugal separator-side part 21b is connected with the branching connector 21f connected with the first tube 25a and has the first pump tube 21g formed in the vicinity of the branching connector 21f.

The second line 22 connecting the outlet 144 of the centrifugal separator 20 and the first line 21 with each other has an end thereof connected to the outlet of the centrifugal separator 20 and the other end thereof connected to the branching connector 21e for connecting the blood collection needle-side part 21a and the second line 22 with each other. The second line 22 has the following elements arranged as viewed from the side of the centrifugal separator 20: a branching connector 22a for connecting the second line 22 with the second tube 25b of the plasma collection bag 25 and with the third tube 26a of the platelet collection bag 26; a branching connector 22c for connecting the second line 22 with a tube having a bubble-removing filter 22f; and a branching connector 22d for connecting the second line 22 with the fourth tube 27a connected with the buffy coat collection bag 27. The third line 23 is connected with the branching connector 21c having an end thereof connected to the first line 21. The third line 23 has the following elements arranged as viewed from the connector 21c: a pump tube 23a; a foreign matter removing filter 23b; a bubble removing chamber 23c; and an anticoagulant container-connecting needle 23d.

The plasma collection bag 25 includes the first tube 25a connected with the branching connector 21f located between the blood collection needle 29 and the pump tube 21g of the first line 21; and the second tube 25b connected with the branching connector 22a of the second line 22. The platelet collection bag 26 includes the third tube 26a connected with the branching connector 22a of the second line 22. The buffy coat collection bag 27 includes the fourth tube 27a connected with the branching connector 22d of the second line 22.

It is preferable to use polyvinyl chloride as the material for the tubes and the pump tubes used to form the first to third lines 21, 22, and 23 and the tubes connected to the bags. Materials similar to those for the tubes can be also used to form the branching connectors. The pump tubes strong enough to bear a pressure applied thereto by roller pumps are used in the embodiment.

Each of the plasma collection bag 25, the platelet collection bag 26, and the buffy coat collection bag 27 is composed of layers of flexible plastic sheets having the peripheral edges thereof fusion bonded (thermally or with high frequencies) or adhered. Soft polyvinyl chloride is the most favorable material for forming the bags 25, 26, and 27.

It is preferable to use a sheet material superior in gas permeability for the platelet collection bag 26 for assuring an improved platelet shelf life.

Essential portions of the platelet collection circuit 2 are housed in a cassette. The platelet collection circuit 2 includes a cassette housing 28 partially accommodating or partially holding all the lines (first, second, and third lines) and all the tubes (first, second, third, and fourth tubes). In other words, they are partially fixed to the cassette housing 28.

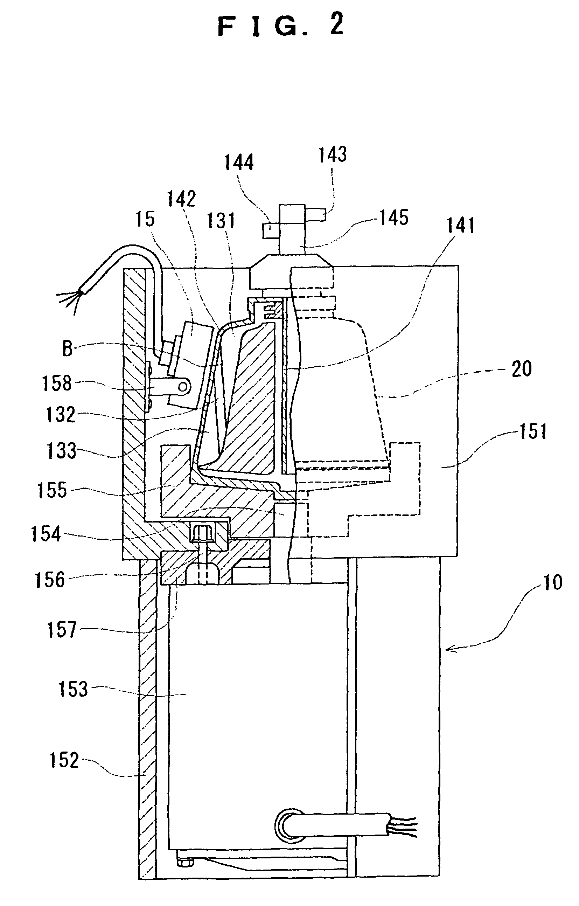

The centrifugal separator 20, a so-called centrifugal bowl, is mounted on the platelet collection circuit 2 to separate blood into components by a centrifugal force. As shown in Fig. 2, the centrifugal separator 20 includes a tubular member 141 extending vertically and having an inlet 143 formed at the upper end thereof and a hollow rotor 142 sealed to prevent liquid from flowing thereinto from an upper portion 145 of the centrifugal separator 20 and rotating around the tubular member 141. The rotor 142 has a flow path (blood storage space) formed along the bottom and the inner peripheral surface thereof. An outlet 144 is so formed as to communicate with the upper portion of the flow path. The volume of the rotor 142 ranges from 100 to 350 ml.

The rotor 142 is rotated under predetermined centrifugal conditions (rotational speed and rotation time) set by the rotor drive unit 10 of the platelet collection apparatus 1. Based on the centrifugal conditions, patterns (for example, the number of blood components to be separated) of blood separation to be made in the rotor 142 can be set. As shown in Fig. 2, according to the embodiment, the centrifugal conditions are set such that the blood is separated into a plasma layer (inner layer) 131, a buffy coat layer (intermediate layer) 132, and a red cell layer (outer layer) 133 laminated in the flow path of the rotor 142.

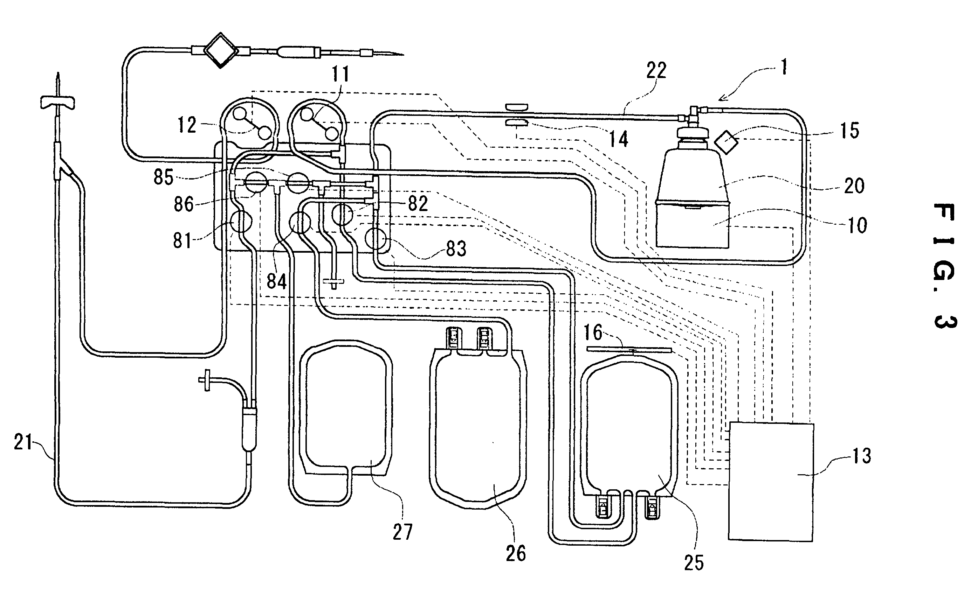

Referring to Fig. 3, the platelet collection apparatus 1 according to the present invention will be described.

The platelet collection apparatus 1 includes the centrifugal separator drive unit 10 for rotating the rotor 142 of the centrifugal separator 20; the first liquid supply pump 11 to be used individually for the first line 21; the second liquid supply pump 12 to be used individually for the third line 23; a plurality of the flow path shutter means 81,82, 83, 84, 85, and 86 for opening/closing the flow paths of the platelet collection circuit 2; and the controller 13 for controlling the centrifugal separator drive unit 10, the first liquid supply pump 11, the second liquid supply pump 12, and the flow path shutter means 81, 82, 83, 84, 85, and 86. The platelet collection apparatus 1 further includes a turbidity sensor 14 mounted on the portion, of the second line 22, located between the centrifugal separator 20 and the connector 22a for connecting the second tube 25b with the second line 22; an optical sensor 15 mounted above the centrifugal separator 20; and a weight sensor 16 for detecting the weight of the plasma collection bag 25.

The turbidity sensor 14 detects the turbidity of a fluid flowing through the second line 22 and outputs a voltage corresponding to the detected turbidity. Specifically, the turbidity sensor 14 outputs a low voltage when the turbidity is high, while it outputs a high voltage when the turbidity is low. As the turbidity sensor, an optical sensor (laser light source, LED light source, and the like) can be preferably used.

In the embodiment, the platelet collection apparatus computes the number of platelet collection cycles and the expected number of to-be-collected platelets or a value related thereto from the data of a donor's hematcrit value, platelet concentration data, and the target number of to-be-collected platelets or a value related thereto, all of which are inputted to the platelet collection apparatus 1 before a platelet collection operation is performed. Therefore the platelet collection apparatus has a hematcrit value input portion 61, a platelet concentration input portion 62, an input portion 63 of the target number of units of to-be-collected platelets related to the target number of to-be-collected platelets.

In the platelet collection apparatus of the present invention, blood to be analyzed is collected from a donor before performing the platelet collection operation, information of blood (hematcrit value, concentration of platelet) is obtained from the blood to be analyzed (preparatorily collected blood) by using a blood analyzer prepared separately. The data of the blood information and the target number of to-be-collected platelets (or target number of units of to-be-collected platelets) are inputted to the platelet collection apparatus to compute a necessary number of cycles of the platelet collection operation. Thereby the platelet collection operation is performed in the number of cycles.

The controller 13 includes a control part 50, a pump controller 53 for the first liquid supply pump 11 and a pump controller 54 for the second liquid supply pump 12, an input part 60, and a display part 52 serving as an warning means. The control part 50 which is the control mechanism of the controller 13 is electrically connected to the first liquid supply pump 11 and the second liquid supply pump 12 through the pump controllers 53 and 54 respectively. The turbidity sensor 14 mounted on the second liquid supply pump 22 is electrically connected to the control part 50. A operational amount detection part 56 mounted on the first liquid supply pump 11 is electrically connected to the control part 50. As the operational amount detection part 56, a rotational amount detection means can be used. More specifically, a rotary encoder can be preferably used as the operational amount detection part 56. The control part 50 is also electrically connected to a driving controller 55 of a driving apparatus (rotor-driving apparatus) for driving the centrifugal separator.

The control part 50 has a memory that stores necessary data, computing equations, and inputted hematcrit value data, and data of the platelet concentration; a function of computing the number of collected platelets (function of computing real time number of collected platelets) by using symbols, related to the platelet concentration, detected by the turbidity sensor; a function of computing the estimated number of to-be-collected platelets; the function of determining on an increase/decrease tendency in the number of collected platelets; the control function for the function of decreasing the number of to-be-collected platelets; and the function of controlling the function of increasing the number of to-be-collected platelets. The platelet collection apparatus of the embodiment is characterized in that it has both the function of decreasing the number of to-be-collected platelets and the function of increasing the number of to-be-collected platelets.

An input part 60 of the controller 13 has a hematcrit value input portion 61, a platelet concentration input portion 62, an input portion 63 of the target number of units of to-be-collected platelets, a start switch 64, and a switch 65 for altering the number of platelet collection cycles.

The platelet collection apparatus 1 computes the number of platelet collection cycles and the expected number of to-be-collected platelets or a value related thereto from the data of the hematcrit value obtained from the blood to be analyzed, the data of the platelet concentration from the blood to be analyzed, and the target number of to-be-collected platelets or a value related thereto. The value related to the target number of to-be-collected platelets means the target number of units of to-be-collected platelets. The value related to the expected number of to-be-collected platelets means the expected number of units of to-be-collected platelets.

The relationship between the number of units of platelets and the number of platelets is as follows:
20-unit platelet: not less than 4×10¹¹
15-unit platelet: not less than 3×10¹¹
10-unit platelet: not less than 2×10¹¹
5-unit platelet: not less than 1×10¹¹
The number of units = 5×number of platelets/10¹¹.
The number of platelets = number of units × 2 × 10¹⁰

Description is made on the function of computing the number of platelet collection cycles and the expected number of to-be-collected platelets or the value related thereto from the data of the hematcrit value obtained from the blood to be analyzed, the data of the platelet concentration from the blood to be analyzed, and the target number of to-be-collected platelets or a value related thereto.

The control part 50 of the platelet collection apparatus of the embodiment computes the number of platelet collection cycles and the expected number of to-be-collected platelets or the value related thereto as follows: In the embodiment, upon input of the target number of units of to-be-collected platelets to the control part 50, the computing portion of the control part 50 computes the target number of to-be-collected platelets.

The expected number of to-be-collected platelets in the first cycle = amount of extracorporeally circulating blood × PLT concentration(platelet concentration) x expected recovery percentage ••• Equation 1

The equation 1 is fundamentally used. In addition, in the second cycle and subsequent cycles thereto, it is necessary to take extracorporeally circulating blood and the amount of blood remaining in the centrifugal bowl into consideration as the amount of treated blood. It is also necessary to consider an increase of the number of platelets owing to buffy coat recycled in the platelet collection apparatus of the embodiment.

The following computation is performed in the second cycle:

The expected number of platelets to be collected in the second cycle = amount of blood to be processed in the second cycle × expected platelet concentration in the second cycle × expected recovery percentage + amount of blood to be processed in the first cycle × expected PLT concentration in the first cycle × left-behind percentage × recycle percentage ••• Equation 2

The following computation is performed in the third cycle:

The expected number of platelets to be collected in the third cycle = amount of blood to be processed in the third cycle × expected platelet concentration in the third cycle × expected recovery percentage + amount of blood to be processed in the second cycle × expected platelet concentration in the second cycle × left-behind percentage × cycle percentage + amount of blood to be processed in the first cycle × expected PLT concentration in the first cycle × left-behind percentage × recycle percentage ••• Equation 3

In sequentially computing the expected number of platelets to be collected in the first cycle, the second cycle, and the third cycle, the number of cycles at which the expected number of platelets exceeds the target number of platelets to be collected is outputted as the number of platelet collection cycles. The number of platelets collected in each cycle is outputted as the expected number of platelets.

The amount of the extracorporeally circulating blood in the equation 1 is found by an equation shown below:

The amount of the extracorporeally circulating blood = K×B(%)÷HCT(%) ••• Equation 4

In the equations 1,2,3, and 4;
K: The volume (200 - 230ml) of red cells inside the centrifugal bowl at the time of termination of blood collection (start of platelet collection operation),
B(%): Percentage (60 - 95%) of the red cells in K,

The value of each of K and B is experimentally found, and a fixed value is used.
HCT(%): hematcrit value (obtained from blood collected for analysis),
PLT concentration: platelet concentration (obtained from the blood collected for analysis)

Expected recovery percentage: the ratio of the number of platelets that can be taken out into the recovery bag as thick platelets from the centrifugal separation container to the total platelets contained in blood supplied to the centrifugal separation container in each cycle. The experience value based on an experiment is used as expected recovery percentage. It is normally 60 ∼ 85%.

Target number of to-be-collected platelets: target number of units of to-be-collected platelets × 2 × 10¹⁰

Expected number of to-be-collected platelets = expected number of platelets to-be-collected in first cycle + expected number of platelets to-be-collected in second cycle + ••• expected number of platelets to-be-collected in last cycle •••• Equation 5

The platelet collection apparatus may compute the expected number of units of to-be-collected platelets. The display part 52 has the function of displaying the number of platelet collection operation cycles and the expected number of to-be-collected platelets or the expected number of units of to-be-collected platelets. From the number of platelet collection operation cycles, the control part 50 may have the function of computing an expected time period required to perform the platelet collection operation. The controller 13 may have the function of displaying an expected time when the platelet collection operation terminates.

The platelet collection apparatus of the present invention has the function of determining whether there is a tendency to increase or decrease in the number of collected platelets by using data, related to the concentration of platelets, obtained from the turbidity sensor, the expected number of to-be-collected platelets or a value related thereto and determining on a "level of tendency to increase" and a "unit-increased level" higher than the "level of tendency to increase" when the plasma (PC) containing high-concentration platelets is collected after blood is collected in a first plasma collection operation, namely, after platelets are collected in the first platelet collection operation is performed, namely, after the plasma containing high-concentration platelets (PC) is collected in a first cycle. The platelet collection apparatus further includes the function of altering an inputted target number of units of to-be-collected platelets in the case where the function of determining on an increase/decrease tendency in the number of collected platelets has determined that the number of units of platelets is on the "unit-increased level". The platelet collection apparatus further includes the function of increasing the amount of platelets to be collected in a subsequent platelet collection operation in the case where the function of altering the target number of units of to-be-collected platelets has altered the target number of units of to-be-collected platelets. The platelet collection apparatus further includes the function of decreasing the amount of platelets to be collected in a subsequent platelet collection operation in the case where the function of altering the target number of units of to-be-collected platelets has not altered the target number of units of platelets in a predetermined period of time after termination of the operation of the function of altering the target number of units of to-be-collected platelets and in the case where the function of determining on an increase/decrease tendency in the number of collected platelets has determined that the number of collected platelets is on the "level of tendency to increase".

The platelet collection apparatus of the embodiment has the function of decreasing the number of to-be-collected platelets and the function of increasing the number of to-be-collected platelets in addition to the function of determining on an increase/decrease tendency in the number of collected platelets.

The function of determining on an increase/decrease tendency in the number of collected platelets will be described in detail below.

The platelet collection apparatus of the embodiment has a expected collection-amount-group determination function of making a determination on to which of stored expected collection-amount-groups, by using the expected number of to-be-collected platelets or the value related thereto; and a function of computing the estimated number of to-be-collected platelets, by using data related to the collection amount of the high-concentration platelets-containing plasma in the platelet collection operation and data related to the platelet concentration obtained from the turbidity sensor when the high-concentration platelets-containing plasma is collected. The function of determining on an increase/decrease tendency in the number of collected platelets determines on an increase tendency by using the data of the corresponding collection-amount-group determined by the collection-amount-group determination function and the estimated number of to-be-collected platelets computed by the function of computing the estimated number of to-be-collected platelets.

The platelet collection apparatus of the embodiment has the collection-amount-group determination function of determining on which of stored expected collection-amount-groups corresponds to the number of collected platelets, by using the computed number of the estimated to-be-collected platelets or the value related thereto. More specifically, the expected collection-amount-groups are classified into a lower group, a medium group, and a higher group. In the lower group, the expected number of to-be-collected platelets is less than a reference value + a first predetermined value (for example, 0.3 × 10¹¹: PM1). The reference value is [target number of units of to-be-collected platelets × 2 × 10¹⁰ - the predetermined value (for example, 0.15 × 10¹¹: PM5)]. In the medium group, the expected number of to-be-collected platelets is not less than the reference value + the first predetermined value (for example, 0.3 × 10¹¹: PM1) nor more than the reference value + a second predetermined value (for example, 0.65 × 10¹¹: PM2) with respect to the reference value. In the higher group, the expected number of to-be-collected platelets is more than the reference value + the second predetermined value (for example, 0.65 × 10¹¹: PM2)). The numerical values of the first predetermined value (PM1), the second predetermined value (PM2), and the predetermined value (PM5) are exemplified for the case where the target number of units of to-be-collected platelets is 10 units and can be altered in dependence on the target number of units of to-be-collected platelets. More specifically, it is preferable to set the values thereof to 1/2 when the target number of units of to-be-collected platelets is five and to 3/2 when the target number of units of to-be-collected platelets is 15.

The platelet collection apparatus of the embodiment has the function of computing the estimated number of to-be-collected platelets, by using data related to the collection amount of the high-concentration platelets-containing plasma in the platelet collection operation and data related to the platelet concentration obtained from the turbidity sensor when the high-concentration platelets-containing plasma is collected.

The platelet collection apparatus of the present invention has a function of computing a real-time number of collected platelets for the function of computing the estimated number of to-be-collected platelets. At the time when the plasma (PC) containing high-concentration platelets is collected, voltage signals detected by the turbidity sensor installed on the second line are sequentially inputted to the control part. The amount of the plasma containing the high-concentration platelet is computed by using an operation amount of the liquid supply pump at the time when the plasma containing high-concentration platelet is collected and a stored blood feeding amount per unit operation amount of the liquid supply pump. The control part stores data or an equation for computing the concentration of platelets in the plasma containing the high-concentration platelet from the voltage signal sent from the turbidity sensor. The control part computes the number of collected platelets (RTM value) from the computed platelet concentration and the computed blood-feeding amount. The number of collected platelets (RTM value) can be obtained by computing the platelet concentration in a predetermined time period on the basis of output signals of the turbidity sensor detected at predetermined intervals (for example, 0.02 - 2 seconds) and multiplying the platelet concentration by the blood-feeding amount in the predetermined time period to thereby obtain the number of collected platelets in each predetermined time period This operation is performed continuously in the time period in which the plasma containing the high-concentration platelet is collected. The total number of the collected platelets can be obtained by adding respective computed number of platelets to each other. The number (RTM value) of the collected platelets can be obtained by computing an average value of a predetermined number of times (for example, 10 times) of output signals of the turbidity sensor detected at predetermined intervals (for example, 0.001 - 0.5 seconds), computing a platelet concentration in a specified time period (for example, 10 times as large as the predetermined time period), and multiplying the platelet concentration by the blood-feeding amount in the specified time period to thereby obtain the number of collected platelets in each predetermined time period. This operation is performed continuously in the time period in which the plasma containing the high-concentration ' platelet is collected. The total number of the collected platelets can be obtained by adding respective computed number of platelets to each other.

The number of collected platelets (RTM value) may be computed without computing the platelet concentration. That is, the number of collected platelets (RTM value) can be computed by using a stored equation for computing the number of collected platelets and data related to the platelet concentration obtained by converting analog data provided by the turbidity sensor into digital data.

An RTM-corrected value is computed from the computed number of collected platelets (RTM value). When the RTM value is not less than the expected number of to-be-collected platelets × 1.5, the RTM-corrected value = the expected number of to-be-collected platelets × 1.2 (case A). When the RTM value is not less than the expected number of to-be-collected platelets × 1.2 nor more than the expected number of to-be-collected platelets × 1.5, the RTM-corrected value = the expected number of to-be-collected platelets × 1.1 (case B). When the RTM value is not less than the expected number of to-be-collected platelets nor more than the expected number of to-be-collected platelets × 1.2, the RTM-corrected value = the expected number of to-be-collected platelets (case C). When the RTM value is less than the expected number of to-be-collected platelets, the RTM-corrected value = RTM value (case D). A display number of collected platelets is also computed. The display number of collected platelets is the sum of the addition of the RTM-corrected values in the first platelet collection operation through the previous platelet collection operation and the RTM-corrected value in the current platelet collection operation. In other words, the display number of collected platelets is the addition of the RTM-corrected values in a terminated platelet collection operation.

The estimated number of to-be-collected platelets is computed by using the corresponding case of the cases A - D and the display number of collected platelets. More specifically, in the case of the cases A, B, and C, the estimated number of to-be-collected platelets is computed by the display number of collected platelets + the RTM-corrected value in the previous platelet collection operation × (set number of platelet collection operation cycles - the number of platelet collection operation cycles when collection operation terminated). In the case of the case D, the estimated number of to-be-collected platelets is computed by the display number of collected platelets + the expected number of to-be-collected platelets (value obtained by dividing the expected number of to-be-collected platelets by the number of cycles) × (set number of platelet collection operation cycles - the number of platelet collection operation cycles when collection operation terminated).

The function of determining on an increase/decrease tendency in the number of collected platelets makes a determination on to which of "level of proper range (no tendency to increase or decrease), "level of tendency to increase", and "unit-increased level" the estimated number of to-be-collected platelets corresponds. The function of determining on an increase/decrease tendency in the number of collected platelets may determine on "level of decrease range" (there is a tendency to decrease).

The function of determining on an increase/decrease tendency in the number of collected platelets determines that the number of collected platelets is on the level of proper range (there is no tendency to increase or decrease) [1] if a reference value □ the estimated number of to-be-collected platelets < the reference value + a second predetermined value (PM2: specifically 0.65 × 10¹¹), supposing that the expected collection-amount-group is "medium" or "high" and [2] if the reference value + the second predetermined value (PM2: specifically 0.65 × 10¹¹) □ the estimated number of to-be-collected platelets < the reference value + a third predetermined value (PM6: specifically 0.9 × 10¹¹), supposing that the expected collection-amount-group is "low".

The function of determining on an increase/decrease tendency in the number of collected platelets determines that the number of collected platelets is on "level of tendency to increase" (there is a tendency to increase) [3] if the reference value + the second predetermined value (PM2: specifically 0.65 × 10¹¹) □ the estimated number of to-be-collected platelets < the reference value + the third predetermined value (PM6: specifically 0.9 × 10¹¹), supposing that the expected collection-amount-group is "medium" or "high" and [4] if the reference value + the third predetermined value (PM6: specifically 0.9 × 10¹¹) □ the estimated number of to-be-collected platelets, supposing that the expected collection-amount-group is "low".

The function of determining on an increase/decrease tendency in the number of collected platelets determines that the number of collected platelets is on "unit-increased level" (there is a tendency to increase) if [5] the reference value + the third predetermined value (PM6: specifically 0.9 × 10¹¹) □ the estimated number of to-be-collected platelets, supposing that the expected collection-amount-group is "high".

In the embodiment, the function of determining on an increase/decrease tendency in the number of collected platelets determines on the number of collected platelets by distinguishing "level of tendency to increase" and "unit-increased level" from each other when there is a tendency to increase. This is because as described later, the platelet collection apparatus has the function of increasing the number of to-be-collected platelets. However, the platelet collection apparatus does not necessarily have to be provided with the function of increasing the number of to-be-collected platelets. In this case, the function of determining on an increase/decrease tendency in the number of collected platelets determines that the number of collected platelets is in the proper range (there is no tendency to increase or decrease) or there is a tendency to increase. In this case, the function of determining on an increase/decrease tendency in the number of collected platelets determines that the number of collected platelets is on the level of proper range (there is no tendency to increase or decrease) [1] if the reference value □ the estimated number of to-be-collected platelets < the reference value + the second predetermined value (PM2: specifically 0.65 × 1011), supposing that the expected collection-amount-group is "medium" or "high" and [2] if the reference value + the second predetermined value (PM2: specifically 0.65 × 10¹¹) □ the estimated number of to-be-collected platelets < the reference value + the third predetermined value (PM6: specifically 0.9 × 10¹¹), supposing that the expected collection-amount-group is "low". The function of determining on an increase/decrease tendency in the number of collected platelets determines that the number of collected platelets is on "level of tendency to increase" (there is a tendency to increase) if [3] the reference value + the second predetermined value (PM2: specifically 0.65 × 10¹¹) □ the estimated number of to-be-collected platelets, supposing that the expected collection-amount-group is "high" and [4] if the reference value + the third predetermined value (PM6: specifically 0.9 × 10¹¹) □ the estimated number of to-be-collected platelets, supposing that the expected collection-amount-group is "medium".

In the case where the function of determining on an increase/decrease tendency in the number of collected platelets has determined that there is no tendency to increase in the number of collected platelets, the platelet collection apparatus performs a platelet collection operation on the basis of stored data of the platelet collection condition.

In the last platelet collection operation, the function of determining on an increase/decrease tendency in the number of collected platelets does not operate. That is, the determination of the tendency in the platelet collection is not made in the last platelet collection operation. However, the display number of collected platelets is computed.

The platelet collection apparatus of the present invention has the function of altering an inputted target number of units of to-be-collected platelets in the case where the function of determining on an increase/decrease tendency in the number of collected platelets has determined that the number of units of collected platelets is on "unit-increased level". The platelet collection apparatus further includes the function of increasing the amount of platelets to be collected in a subsequent platelet collection operation in the case where the function of altering an inputted target number of units of to-be-collected platelets has altered the target number of units of to-be-collected platelets.

The platelet collection apparatus has a display function of giving warning of "unit-increased level" operates when the function of determining on an increase/decrease tendency in the number of collected platelets has determined that the number of units of collected platelets is on the increase level. At this time, the function of altering the target number of units of to-be-collected platelets also operates. Hence it is possible to alter the inputted target number of units of to-be-collected platelets. The target number of units of to-be-collected platelets is rewritten not automatically. An altered number of units can be inputted by an operator. The alteration of the number of units is inputted from the input portion 63 of the target number of units of to-be-collected platelets. The "unit-increased increase level" is displayed on the display part 52.

When the target number of units of to-be-collected platelets is altered, the warning display of "unit-increased level" is turned off, and the control part 50 computes and rewrites a necessary platelet collection condition for collecting the target number of units of platelets and executes subsequent platelet collection operations under a new platelet collection condition. Specifically, the control part 50 computes and rewrites the target collection amount of plasma containing high-concentration platelet and a target collection amount of platelet-removed of plasma. It is preferable not to alter the total amount of plasma to be collected from a donor. Because of the increase of the target number of units of to-be-collected platelets, the target collection amount of the plasma containing high-concentration platelets has increased. Thus the target collection amount of platelet-removed of plasma is decreased by the increased amount of the target collection amount of the plasma containing high-concentration platelets.

When the function of altering an inputted target number of units of to-be-collected platelets has altered the target number of units of to-be-collected platelets, the computing portion of the control part 50 re-computes the expected number of to-be-collected platelets or the value related thereto. The function of determining on an increase tendency in the number of collected platelets makes a determination by using the re-computed expected number of to-be-collected platelets or the value related thereto.

The platelet collection apparatus has a function of computing a re-computed expected number of to-be-collected platelets or a value related thereto when the function of altering an inputted target number of units of to-be-collected platelets alters a target number of units of to-be-collected platelets. The function of determining on an increase tendency in the number of collected platelets makes a determination by using a re-computed expected number of to-be-collected platelets or a value related thereto.

The platelet collection apparatus has the function of decreasing the amount of platelets to be collected in a subsequent platelet collection operation in the case where the function of altering the target number of units of to-be-collected platelets has not altered the target number of units of platelets in a predetermined period of time after termination of the operation of the function of altering the target number of units of to-be-collected platelets.

The platelet collection apparatus has the function of decreasing the amount of platelets to be collected in a subsequent platelet collection operation in the case where the function of determining on an increase/decrease tendency in the number of collected platelets has determined that the number of collected platelets is on the "level of tendency to increase".

That is, the platelet collection apparatus of the embodiment has the function of decreasing the number of to-be-collected platelets operates in the case where the target number of units of to-be-collected platelets has not been altered in a predetermined period of time after the function of altering an inputted target number of units of to-be-collected platelets has start to operate and in the case where the function of determining on an increase tendency in the number of collected platelets has determined that the number of collected platelets is on the "level of tendency to increase".

The function of decreasing the number of to-be-collected platelets alters the stored data of the platelet collection termination condition to quicken the platelet collection operation and decrease the collection amount of the plasma containing high-concentration platelets (type 1 of the function of decreasing the number of to-be-collected platelets). The function of decreasing the number of to-be-collected platelets may alter the stored data of the platelet collection condition to decrease the concentration of platelets contained in the plasma containing the high-concentration platelets (type 2 of the function of decreasing the number of to-be-collected platelets).

Particularly, the platelet collection apparatus of the embodiment has both types 1 and 2.

More specifically, the platelet collection apparatus performs the platelet collection operation at least three times. In the platelet collection operation except the last platelet collection operation, the function of decreasing the number of to-be-collected platelets alters the stored data of the platelet collection termination condition to quicken the platelet collection operation and decrease the collection amount of the plasma containing high-concentration platelets. In the last platelet collection operation, the function of decreasing the number of to-be-collected platelets alters the stored data of the platelet collection condition to decrease the concentration of platelets contained in the plasma containing the high-concentration platelets.

The decrease type 1 of the function of decreasing the number of to-be-collected platelets alters the stored data of the platelet collection termination condition by decreasing the voltage of the collection termination turbidity sensor by a predetermined value to quicken the platelet collection operation. That is, the type 1 alters a platelet concentration (for example, about 400,000/ml) in a normal platelet collection termination to a platelet concentration (for example, about 900,000/ml) in an adjusted platelet collection termination. Thereby the time period for collecting the plasma containing the high-concentration platelets becomes short. Thus the collection amount of the plasma containing the high-concentration platelets becomes smaller. This type of adjustment can be simply and reliably accomplished. However, the amount of the plasma containing the high-concentration platelets decreases.

The decrease type 2 of the function of decreasing the number of to-be-collected platelets alters the stored data of the platelet collection termination condition in such a way that the concentration of platelets contained in the plasma containing the high-concentration platelets decreases, i.e., in such a way that the collection amount of the plasma is equal to or larger than that at the time when the adjusting function does not operate and that the platelet concentration decreases. More specifically, when the plasma containing high-concentration platelets is collected, the type 2 decreases the collection speed of the plasma containing the high-concentration platelets to a predetermined level after platelets flow out therefrom and increase the voltage of the collection termination turbidity sensor by a predetermined value to delay the platelet collection operation. That is, the type 2 alters a platelet concentration (for example, about 400,000/ml) in the normal collection termination to a platelet concentration (for example, about 300,000/ml) in the adjusted collection termination. Thereby the degree of the concentration platelets in the plasma decreases a little, and the collection amount thereof is more than that collected in the normal condition. By operating the decrease type 2 in the last platelet collection operation, the amount of the plasma containing the high-concentration platelets is almost equal to that collected in the normal condition.

It is preferable to provide both the type 1 and the type 2. However, one of the type 1 and the type 2 may be provided.

It is preferable that the platelet collection apparatus has an automatic function of terminating the operation of the function of decreasing the number of to-be-collected platelets and performing a platelet collection operation in accordance with data of the platelet collection condition which is used before the function of decreasing the number of to-be-collected platelets operates, when the function of determining on an increase tendency in the number of collected platelets has determined that the number of collected platelets has "there is no tendency to increase" after the function of decreasing the number of to-be-collected platelets operates. By doing so, it is possible to prevent an excessive reduction in the collection of the number of platelets which is caused by the operation of the function of decreasing the number of to-be-collected platelets.

In the platelet collection apparatus of the embodiment, the determination on the increase tendency in the number of collected platelets is continued after the function of decreasing the number of to-be-collected platelets starts its operation. Unlike the operation to be made after the function of increasing the number of to-be-collected platelets operates, the determination on the increase tendency in the number of collected platelets is made in accordance with the initial condition without altering the determination condition. In other words, the operation of the function of decreasing the number of to-be-collected platelets is effective only when a subsequent platelet collection operation is made, whereas the determination on the increase tendency in the number of to-be-collected platelets is executed in each platelet collection operation. Therefore when the function of decreasing the number of to-be-collected platelets operates because determination of "level of tendency to increase" and "unit-increased level" has been made in the first platelet collection operation, the function of decreasing the number of to-be-collected platelets does not operate if the determination of "level of proper range" has been made by the function of determining on an increase tendency in the number of collected platelets in the next platelet collection operation.

The platelet collection apparatus has a warning function that operates when it has been determined that there is a tendency to increase in the number of collected platelets and an operation display function of informing an operator of the operation of the function of decreasing the number of to-be-collected platelets.

In addition to the automatic type, the function of decreasing the number of to-be-collected platelets may be actuated by an operator's switching operation.

The platelet collection apparatus of the present invention is described below in other words. It is preferable that the platelet collection apparatus has the function of automatically computing the total number of platelets including the estimated number of platelets to be collected in remaining operation cycles after the operation cycle of each platelet collection operation terminates and the number of platelets collected in each cycle is automatically computed, has the function of determining on which of the four stages, namely, "level of tendency to decrease", "level of proper range", "level of tendency to increase" and "unit-increased level" corresponds to the number of to-be-collected platelets, and has the function of executing remaining operation cycles in dependence on each case of the four stages.

There is a case where the number of collected platelets corresponds to the case of "level of tendency to decrease". Thus it is preferable that the platelet collection apparatus has a function of informing the operator of the "level of tendency to decrease" by making a warning display such as "collected amount is short" and/or giving warning sound and automatically adjusting factors such as a collection amount of blood in the next cycle, a blood collection speed, a centrifuging separation speed, a plasma circulation speed for collection of platelets, and a platelet collection termination timing affecting the collection of platelets to increase the number of to-be-collected platelets. The platelet collection apparatus has a function of continuing an operation condition to be applied to the start of the blood collection operation when the tendency in the number of collected platelets is on the "level of proper range". When the tendency in the number of collected platelets corresponds to the case of "level of tendency to increase", it is preferable that the platelet collection apparatus has a function of informing the operator of that by making a warning display "collected amount is excessive" and/or giving warning sound so that the information allows the operator to issue an instruction of "prevent excessive collection" and automatically adjusting factors such as the collection amount of blood in the next cycle, the blood collection speed, the centrifuging separation speed, the plasma circulation speed for collection of platelets, and the platelet collection termination timing affecting the collection of platelets to decrease the collection the number of to-be-collected platelets. When the tendency in the number of collected platelets corresponds to the case of "unit-increased level", it is preferable to inform the operator of that by making a warning display "unit has increased" and/or giving a warning sound so that the information allows the operator to issue an instruction of "prevent excessive collection" and also issue an instruction of "unit has increased". In issuing the instruction of "prevent excessive collection", it is preferable that the platelet collection apparatus has the function of automatically adjusting factors such as the collection amount of blood in the next cycle, the blood collection speed, the centrifuging separation speed, the plasma circulation speed for collection of platelets, and the platelet collection termination timing affecting the collection of platelets to decrease the number of to-be-collected platelets. In issuing the instruction of "unit has increased", it is preferable that the platelet collection apparatus has the function of automatically altering the collection condition such as the target number of units of to-be-collected platelets, the collection amount of platelets, and the collection amount of plasma to values corresponding to the target number of units of to-be-collected platelets and the function of automatically releasing the warning of "unit has increased".

The controller 13 has a mechanism constructed of a microcomputer and a function of computing the number of rotations of the rotor. Detection signals outputted from the weight sensor 16, the optical sensor 15, and the turbidity sensor 14 are applied to the controller 13. The controller 13 controls the start and stop of the rotation and rotational direction (forward/backward) of each pump, based on the signals applied thereto from the turbidity sensor 14, the optical sensor 15, and the weight sensor 16. The controller 13 also controls the opening/closing of the flow path shutter means 81, 82, 83, 84, 85, and 86 and the operation (rotation of rotor) of the centrifugal separator drive unit 10.

The first flow path shutter means 81 is used to open/close the portion of the first line 21 located between the blood collection needle 29 and the pump tube 21g. The second flow path shutter means 82 is used to open/close the first tube 25a of the plasma collection bag 25. The third flow path shutter means 83 is used to open/close the second tube 25b of the plasma collection bag 25. The fourth flow path shutter means 84 is used to open/close the third tube 26a of the platelet collection bag 26. The fifth flow path shutter means 85 is used to open/close the portion of the second line 22 located between the centrifugal separator 20 and the connector 22d for connecting the second line 22 with the fourth tube 27a of the buffy coat collection bag 27.

The sixth flow path shutter means 86 is used to open/close the portion of the second line 22 located downstream from the connector 21e for connecting the first line 21 and the second line 22 with each other to the connector 22d for connecting the fourth tube 27a of the buffy coat collection bag 27 and the second line 22 with each other. Each of the flow path shutter means 81 through 86 has an insertion portion on which the lines or the tubes are installed. The insertion portion has a clamp to be operated by a drive source such as a solenoid, an electric motor, and a hydraulic or pneumatic cylinder. A clamp to be operated by a hydraulic pressure cylinder is preferable. The clamp of each of the flow path shutter means 81 through 86 is operated based on a signal transmitted thereto from the controller 13.

As shown in Fig. 2, the rotor drive unit 10 includes a rotor drive unit housing 151 accommodating the centrifugal separator 20, a pedestal 152, a motor 153 constituting the drive source, and a disk-shaped rest 155 for holding the centrifugal separator 20. The housing 151 is fixedly mounted on the pedestal 152. The motor 153 is fixed to the lower surface of the housing 151 with a bolt 156 via a spacer 157. The upper end of the rotary shaft 154 of the motor 153 is fitted in the rest 155 such that the rest 155 rotates coaxially and integrally with the rotational shaft 154. The upper portion of the rest 155 has a recess formed therein into which the bottom of the rotor 142 is fitted. The upper portion 145 of the centrifugal separator 20 is fixed to the housing 151 with a fixing member not shown. Once the motor 153 for the rotor drive unit 10 is started, the rest 155 and the rotor 142 fixed thereto are rotated at a rotational speed ranging from 3000 to 6000 rpm.

The rotor drive unit housing 151 has the optical sensor 15 fixedly mounted on an inner wall thereof with a mounting member 158. The optical sensor 15 optically detects the boundaries between separated blood components (for example, the interface B between the plasma layer 131 and the buffy coat layer 132 and the interface between the buffy coat layer 132 and the red cell layer 133) in the centrifugal separator 20. The optical sensor 15 is of a type capable of vertically scanning the peripheral surface of the rotor 142. The optical sensor 15 includes a light source for emitting a light beam to a shoulder portion of the centrifugal separator 20 and a light-receiving part for receiving a light beam reflected by the centrifugal separator 20 and returned therefrom. That is, a light emitting element such as an LED or laser and a light-receiving element are arrayed in a row. A light beam emitted by the light emitting element and reflected by a blood component is received by the light-receiving element, and the amount of the received light is photoelectrically converted.

Because the intensity of the reflected light varies depending on the separated blood component (for example, the interface B between the plasma layer 131 and the buffy coat layer 132), a position of the light-receiving element at which the amount of received light has changed is detected as the corresponding position of the interface B. More specifically, the arrival of the buffy coat layer at a light passage portion is detected, based on the difference between the amount of received light at the time when the light passage portion of the centrifugal separator 20 is filled with a transparent liquid (plasma or water) and the amount of received light at the time when the light passage portion thereof is filled with the buffy coat layer. The detection position of the buffy coat layer is adjusted by changing a light passage position in the centrifugal separator 20. Normally, the detection of the light passage position is unchangeably set.

The turbidity sensor 14 detects the turbidity of a fluid flowing through the second line 22 and outputs a voltage corresponding to the detected turbidity. Specifically, the turbidity sensor 14 outputs a low voltage when the turbidity is high, while it outputs a high voltage when the turbidity is low.

As each of the first liquid supply pump 11 on which the pump tube 21g of the first line 21 is mounted and the second liquid supply pump 12 on which the pump tube 23a of the third line 23 is mounted, a roller pump or a peristaltic pump which is kept out of contact with the blood is preferably used. A pump that can supply the blood in either direction is used as the first liquid supply pump 11 (blood pump). A roller pump capable of rotating forward/ backward is used.

Under the control of the controller, the following steps are executed: a plasma collection/plasma circulation step, to be executed at least one time, consisting of a plasma collection step for collecting anticoagulant-added blood, separating collected blood, and collecting separated plasma into the plasma collection bag and a plasma circulation step for circulating the plasma contained in the plasma collection bag by the plasma collection step to the centrifugal separator; a platelet collection step for flowing out platelets from the centrifugal separator by accelerating a plasma circulation speed by means of the first liquid supply pump after the plasma collection/plasma circulation step is executed and collecting platelets into the platelet collection bag; and a blood return step for returning the blood inside the centrifugal separator to a donor after the platelet collection step is executed.

The controller 13 of the platelet collection apparatus 1 according to the embodiment controls the centrifugal separator drive unit 10, the first liquid supply pump 11, the second liquid supply pump 12, and a plurality of the flow path shutter means to execute at least twice of the platelet collection operation including the plasma collection/constant-speed circulation step, the plasma collection/acceleration plasma circulation step, the platelet collection step, and the blood return step.

Subsequent to the termination of the execution of a platelet collection step and prior to the start of the execution of a blood return step, the controller 13 of the platelet collection apparatus 1 executes a buffy coat-collection step of flowing out the buffy coat from the centrifugal separator 20 and collecting it into the buffy coat collection bag 27 by setting a plasma circulation rate higher than a final plasma circulation rate in the platelet collection step by means of the first liquid supply pump 11.

After the execution of the buffy coat collection step terminates and before the execution of the subsequent blood collection step starts, the controller 13 controls the first liquid supply pump 11 and a plurality of the flow path shutter means 81 through 86 to execute the huffy coat return step of returning the collected huffy coat to the centrifugal separator 20.

The platelet collection operation includes a plasma collection/ circulation (preferably, constant-speed circulation) operation, the platelet collection operation (in other words, plasma containing high-concentration platelets collection operation or high-concentration platelets-containing plasma collection operation) after a plasma collection/circulation operation, and a blood return operation after the platelet collection operation. The platelet collection operation may include a plasma collection/acceleration plasma circulation operation after the plasma collection/constant-speed circulation operation and before the platelet collection operation (in other words, plasma containing high-concentration platelets collection operation).

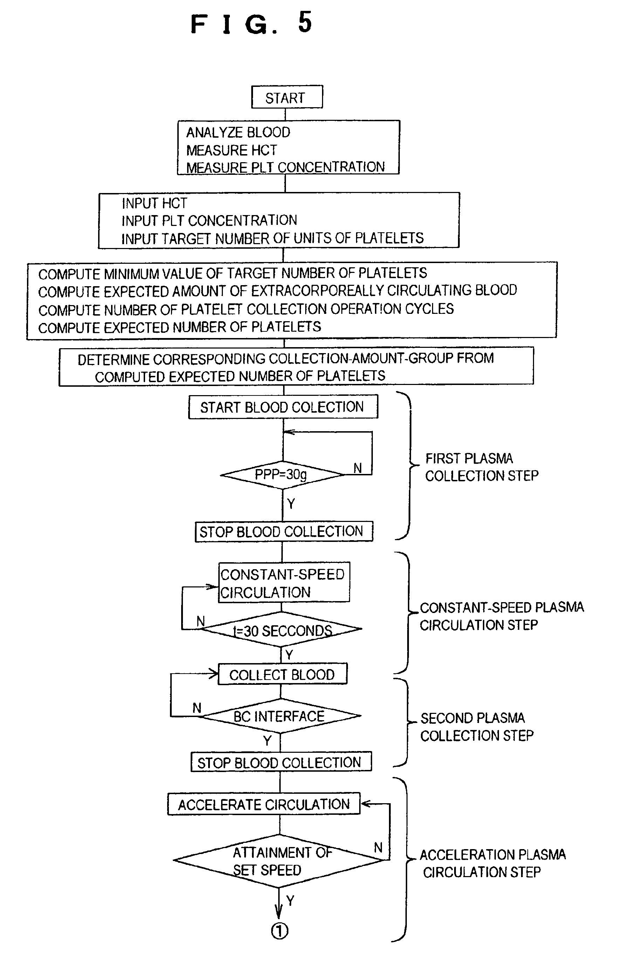

The platelet collection operation will be described below with reference to Figs. 5 through 13.

As shown in Fig. 5, initially, blood cell components are measured beforehand by using sampling blood collected from the donor immediately before the operator starts to collect the components of the blood. A hematcrit value (HCT) of the donor, a platelet concentration (PLT concentration) measured at this time, and the target number of units of to-be-collected platelets are inputted to the platelet collection apparatus. The control part of the platelet collection apparatus computes a minimum value of the target number of to-be-collected platelets, the amount of extracorporeally circulating blood, the number of platelet collection operation cycles, and the expected number of to-be-collected platelets by using the above-described inputted values and data or computing equations stored therein. The control part may compute the expected number of units of to-be-collected platelets. Thereafter the number of platelet collection operation cycles and the expected number of to-be-collected platelets or the expected number of units of to-be-collected platelets are displayed on the display part 52. The control part 50 may have the function of computing an expected operation termination time, based on the number of platelet collection operation cycles and displaying the results. Then the control part makes a determination on to which of the stored expected three collection-amount-groups the computed expected value of the number of to-be-collected platelets corresponds. In other words, a group determination is executed. In the embodiment, the control part determines on to which of the three collection-amount-groups "low", "medium", and "high" the computed expected number of to-be-collected platelets, corresponds and results are stored.

The expected number of to-be-collected platelets is computed by the following equation 5: The expected number of to-be-collected platelets = expected number of platelets to-be-collecled in first cycle + expected number of platelets to-be-collected in second cycle + ••• expected number of platelets to-be-collected in last cycle •••• Equation 5

The expected number of platelets to-be-collected in the first cycle is computed by the above-described equation 1. The expected number of platelets to-be-collected in the second cycle is computed by the above-described equation 2. The expected number of platelets to-be-collected in the third cycle is computed by the above-described equation 3.

The expected recovery percentage is 65 - 80%. The target number of to-be-collected platelets is computed from the target number of units of platelets × 2 × 10¹⁰.

First, the third line 23 and the blood collection needle 29 are primed with the anticoagulant. Then the blood collection needle 29 is stuck on the donor.

As shown in Fig. 5, the controller 13 executes the first plasma collection step of collecting the anticoagulant-added blood by starting the first and second liquid supply pumps 11, 12 and collecting the first predetermined amount of the plasma into the plasma collection bag 25 by activating the centrifugal separator drive unit 10.

When the first blood collection starts, the blood pump 11 is rotated at a predetermined rate (for example, 60 ml/min). At this time, a second pump constituting the anticoagulant pump supplies the anticoagulant (for example, ACD-A solution) at a predetermined rate (for example, 1/10 of the blood pump rate). The blood drawn from the donor is mixed with the ACD-A solution, flows through the first line 21, the chamber, and the first flow path shutter means 81, and flows into the centrifugal separator 20. At the above time point, the sixth flow path shutter means 86, the fifth flow path shutter means 85, the second flow path shutter means 82, and the third flow path shutter means 83 are dosed. Meanwhile the first flow path shutter means 81 and the fourth flow path shutter means 84 are open. When the ACD-A solution-added blood is fed to the centrifugal separator 20, sterilized air that has stayed in the centrifugal separator 20 flows into the platelet collection bag 26 through the second line 22 and the flow path shutter means 84.

Simultaneously with the start of the blood collection, the centrifugal separator 20 starts rotating at a predetermined speed while it is being supplied with the ACD-A solution-added blood. In the centrifugal separator 20, therefore, the blood is centrifugally separated into a plasma layer (inner layer), a buffy coat layer (BC layer, intermediate layer), and a red cell layer (outer layer). When the ACD-A solution-added blood (about 270 ml) is supplied in excess of the capacity of the centrifugal separator 20, the centrifugal separator 20 is completely filled with the blood, and the plasma overflows through the outlet of the centrifugal separator 20. The turbidity sensor 14 mounted on the second line 22 connected with the outlet of the centrifugal separator 20 detects that the fluid flowing through the second line 22 has changed from the air to the plasma. Upon receipt of the detection signal transmitted from the turbidity sensor 14, the controller 13 closes the fourth flow path shutter means 84 and opens the third flow path shutter means 83 to collect the plasma into the plasma collection bag 25.

The weight of the plasma collection bag 25 is measured in advance by the weight sensor 16, and a signal indicating the measured weight is transmitted to the controller 13. Thus, when the weight of the plasma collected in the plasma collection bag 25 is increased to a predetermined amount (for example, 10 to 150g, or 30g), the controller 13 doses the first flow path shutter means 81 and opens the second flow path shutter means 82 to proceed to the constant-speed plasma circulation step.

In the constant-speed circulation step, the blood collection is temporarily suspended, and the centrifugal separator drive unit 10 is activating. The plasma contained in the plasma collection bag 25 is circulated to the centrifugal separator 20 at a constant speed.

Upon start of the execution of the constant-speed circulation step, the controller 13 keeps the first flow path shutter means 81 dosed and the second flow path shutter means 82 opened, stops the ACD pump 12, and starts the blood pump 11 at the predetermined rate (60 to 250 ml/min, for example, 200 ml/min). The plasma collected in the plasma collection bag 25 is fed to the centrifugal separator 20 rotating at a predetermined speed (computed initial number of rotations of rotor) through the second flow path shutter means 82. At the same time, the plasma which has flowed from the centrifugal separator 20 flows into the plasma collection bag 25 through the turbidity sensor 14 and the third flow path shutter means 83. With the lapse of a predetermined length of time (10 to 90 sec, for example, 30 seconds) from the start of the constant-speed circulation step, the controller 13 closes the second flow path shutter means 82 and opens the first flow path shutter means 81 to proceed to the second plasma collection step. The first plasma circulation step is executed preferably at the flow rate of 60 ml/min or more for at least 10 seconds.

In the second plasma collection step, the first and second liquid supply pumps 11, 12 are started to collect the anticoagulant-added blood. Upon detection of the buffy coat layer in the centrifugal separator 20 because of an increase in the amount of the plasma in the platelet collection bag 26, a detection signal is transmitted from the optical sensor 15 to the controller 13. Upon receipt of the signal, the controller 13 doses the first flow path shutter means 81 and opens the second flow path shutter means 82 to proceed to the acceleration plasma circulation step.

More specifically, the first blood pump 11 is activated to collect the blood at a predetermined rate (for example, 60 ml/min). At this time, the second pump constituting the anticoagulant pump supplies the anticoagulant (for example, ACD-A solution) at a predetermined rate (for example, 1/10 of the rate of the blood pump).

The blood drawn from the donor is mixed with the ACD-A solution. The ACD-A solution-added blood flows into the centrifugal separator 20 rotating at a predetermined rate (computed initial number of rotations of rotor), and the plasma is collected in the plasma collection bag 25. Normally, upon detection of the buffy coat layer in the centrifugal separator 20 because of an increase in the amount of the plasma in the platelet collection bag 26, a detection signal is transmitted from the optical sensor 15 to the controller 13. Upon receipt of the signal, the controller 13 doses the first flow path shutter means 81 and opens the second flow path shutter means 82 to proceed to the acceleration plasma circulation step. In the second plasma collection step, the plasma is collected until the optical sensor 15 detects the buffy coat (BC interface: interface between plasma layer and buffy coat layer).

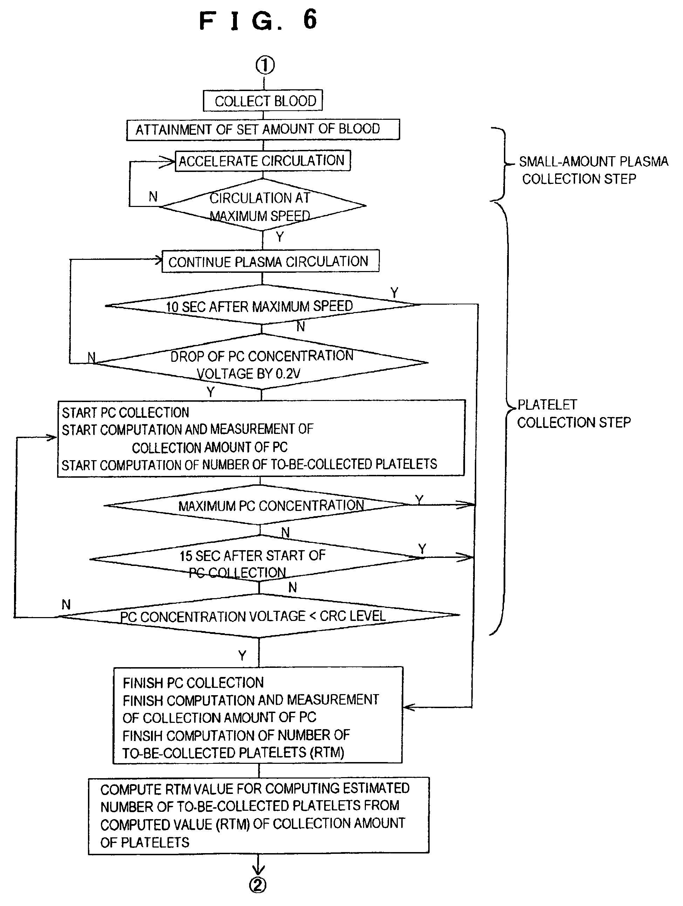

In the acceleration plasma circulation step, the blood collection is temporarily suspended and the centrifugal separator drive unit 10 is activated to circulate the plasma in the plasma collection bag 25 to the centrifugal separator 20. In the processing, the blood pump operates at an initial rate, for example, at 60 ml/min lower than that in the constant-speed plasma circulation step and accelerated until a final rate reaches 150 ∼ 200 ml/min. The acceleration of the pomp is carried out at 2 - 10ml/min for 14 - 70 seconds until a final rate reaches 200 ml/min. Upon completion of the acceleration plasma circulation step, the processing proceeds to ① of the flowchart of Fig. 6 showing the step of collecting a small amount of plasma for interface adjustment.

As shown in Fig. 6, in the small-amount blood collection step for interface adjustment, to keep the position of the buffy coat layer constant, irrespective of donors in the subsequent platelet collection step, the blood is collected in correspondence to a predetermined amount of red cells to be supplied. The supply amount of the red cells is defined as a value derived from dividing a collected amount of blood by the hematocrit value of the donor. Practically about 12 ml of the blood is collected. The blood collection starts by rotating the first liquid supply pump 11 at a predetermined rate (for example, 60 ml/min). At this time, the second pump constituting the anticoagulant pump also supplies the anticoagulant (such as the ACD-A solution) at a predetermined rate (for example, 1/10 of the blood pump rate). The blood collected from the donor is mixed with the ACD-A solution. The ACD-A solution-added blood flows into the centrifugal separator 20 rotating at a predetermined rate (computed initial number of rotations of rotor), and a small amount of plasma is collected. Based on a set collection amount and a set pump rotation rate, the controller 13 computes a blood collection time, and terminates the blood collection when the computed blood collection time expires. Then the controller 13 closes the first flow path shutter means 81 and opens the second flow path shutter means 82 to proceed to the platelet collection step.

Upon termination of the acceleration plasma circulation step, the controller 13 executes the platelet collection step of flowing out platelets from the centrifugal separator 20 and collecting them into the platelet collection bag 26 by accelerating the plasma circulation rate by means of the first liquid supply pump 11. The platelet collection step is called acceleration processing. In this step, the controller 13 controls the rotational speed of the blood pump to increase it stepwise by 10 ml/min every predetermined time period (for example, every one second) from 60 ml/min to 200 ml/min. When the rotational speed of the blood pump reaches 200 ml/min, it is maintained until the platelet collection step terminates.

Upon start of the platelet collection step, the turbidity sensor 14 detects the turbidity of a liquid passing therethrough and outputs a voltage corresponding to a detected turbidity. The output signal of the turbidity sensor 14 is applied to the controller 13. When the rotational speed of the blood pump increases and reaches a rate ranging from 160 to 200 ml/min, platelets contained in the buffy coat layer that have stayed in the centrifugal separator 20 flow out therefrom. As a result of the release of the platelets, the turbidiy of the liquid passing through the turbidity sensor 14 becomes high. At a time point when a voltage outputted from the turbidity sensor 14 drops by 0.2 V, the third flow path shutter means 83 is closed and the fourth flow path shutter means 84 is opened. Thus the platelet-rich plasma released from the centrifugal separator 20 is collected in the platelet collection bag 26. The controller 13 converts the voltage outputted from the turbidity sensor 14 into a platelet concentration to compute the number of platelets collected in the platelet collection bag 26 by driving the liquid supply pump. The concentration of high-concentration platelets-containing plasma passing through the turbidity sensor drops after reaching a maximum value. At a time point when it is detected that a termination level (CRC level) is attained, the execution of the platelet collection step finishes and the processing proceeds to the buffy coat collection step.

As shown in Fig. 6, simultaneously with the start of the collection of the high-concentration platelets-containing plasma (PC), computation and measurement of the collection amount of the high-concentration platelets-containing plasma and computation of the number of collected platelets start. Voltage signals detected by the turbidity sensor are sequentially inputted to the control part. The amount of the plasma containing the high-concentration platelet is computed by using an operation amount of the liquid supply pump and a stored blood feeding amount per unit operation amount. The control part computes the number of collected platelets (RTM value) from the platelet concentration computed by using the voltage signal sent from the turbiditv sensor and a computed blood-feeding amount computed as described above and displays the number of collected platelets. The number of collected platelets (RTM value) can be obtained by computing the platelet concentration in a predetermined time period from output signals of the turbidity sensor detected at predetermined intervals (for example, 0.01 - 2 seconds) and multiplying the platelet concentration by the blood-feeding amount in the predetermined time period to thereby obtain the number of collected platelets in each predetermined time period. This operation is performed continuously in the time period in which the plasma containing the high-concentration platelet is collected The total number of the collected platelets can be obtained by adding respective computed number of platelets to each other. The RTM-corrected value is computed from the computed number of collected platelets (RTM value). When the RTM value is not less than the expected number of to-be-collected platelets × 1.5, the RTM-corrected value = the expected number of to-be-collected platelets × 1.2 (case A). When the RTM value is not less than the expected number of to-be-collected platelets × 1.2 nor more than the expected number of to-be-collected platelets × 1.5, the RTM-corrected value = the expected number of to-be-collected platelets × 1.1 (case B). When the RTM value is not less than the expected number of to-be-collected platelets nor more than the expected number of to-be-collected platelets × 1.2, the RTM-corrected value = the expected number of to-be-collected platelets (case C). When the RTM value is less than the expected number of to-be-collected platelets, the RTM-corrected value = RTM value (case D). The control part computes display number of collected platelets which is the sum of the addition of the RTM-corrected values in an initial platelet collection operation through a previous platelet collection operation and the RTM corrected value in a current platelet collection operation.

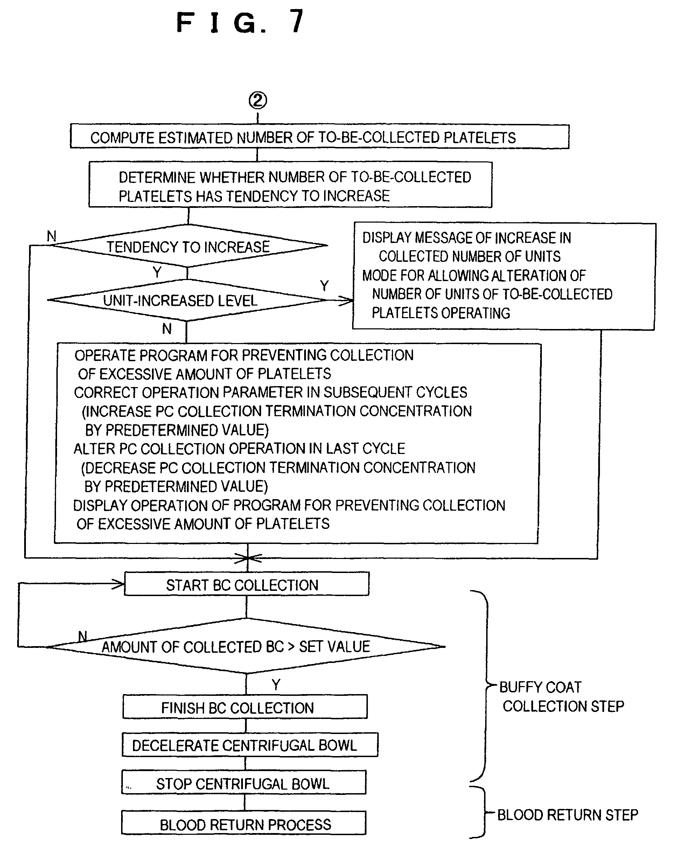

Then the processing proceeds to ② of Fig. 7 in which the estimated number of to-be-collected platelets is computed by using the corresponding case of the cases A - D and the display number of collected platelets. More specifically, in the case of the cases A, B, and C, the estimated number of to-be-collected platelets is computed by the display number of collected platelets + the RTM-corrected value in the previous platelet collection operation × (set number of platelet collection operation cycles - the number of platelet collection operation cycles when collection operation terminated). In the case of the case D, the estimated number of to-be-collected platelets is computed by the display number of collected platelets + the expected number of to-be-collected platelets × (set number of platelet collection operation cycles - the number of platelet collection operation cycles when collection operation terminated).

The function of determining on an increase/decrease tendency in the number of collected platelets determines whether there is a tendency to increase in the number of collected platelets. As shown in Fig. 7, the function of determining on an increase/decrease tendency in the number of collected platelets determines on to which of the three stages, namely, "there is no tendency to increase (level of proper range)", "level of tendency to increase", and "unit-increased level" the estimated number of collected platelets corresponds. More specifically, the function of determining on an increase/decrease tendency in the number of collected platelets determines on whether the number of collected platelets has a tendency to increase or decrease, based on the estimated number of platelets and corresponding groups ("low", "medium", and "high") determined from the above-described computed value of the expected number of platelets. More specifically, the function of determining on an increase/decrease tendency in the number of collected platelets determines that the number of collected platelets is on the proper range level (there is no tendency to increase or decrease) [1] if a reference value □ the estimated number of to-be-collected platelets < the reference value + a second predetermined value (PM2: specifically 0.65 × 10¹¹), supposing that the expected collection-amount-group is "medium" or "high" and [2] if the reference value + the second predetermined value (PM2: specifically 0.65 × 10¹¹) □ the estimated number of to-be-collected platelets < the reference value + a third predetermined value (PM6: specifically 0.9 × 10¹¹), supposing that the expected collection-amount-group is "low". The function of determining on an increase/decrease tendency in the number of collected platelets determines that the number of collected platelets is on "level of tendency to increase" (there is a tendency to increase) if [3] the reference value + the second predetermined value (PM2: specifically 0.65 × 10¹¹) □ the estimated number of to-be-collected platelets < the reference value + the third predetermined value (PM6: specifically 0.9 × 10¹¹), supposing that the expected collection-amount-group is "high" and [4] if the reference value + the third predetermined value (PM6: specifically 0.9 × 10¹¹) □ the estimated number of to-be-collected platelets, supposing that the expected collection-amount-group is "medium". The function of determining on an increase/decrease tendency in the number of collected platelets determines that the number of collected platelets is on "unit-increased level" (there is a tendency to increase) if [5] the reference value + the third predetermined value (PM6: specifically 0.9 × 10¹¹) □ the estimated number of to-be-collected platelets, supposing that the expected collection-amount-group is "high".

If it is determined that there is "no tendency to increase (level of proper range)" in the number of collected platelets, the collection condition is not changed and the processing proceeds to a buffy coat collection step. If it is determined that the number of collected platelets has the "tendency to increase", whether the number of collected platelets is on the "unit-increased level" is determined. If it is determined that the number of collected platelets is not on the "unit-increased level", it is determined that the number of collected platelets is on the "level of tendency to increase". As a result, a program for preventing collection of an excessive amount of platelets (function of decreasing the number of to-be-collected platelets) operates, which is displayed on the display part 52. When the program for preventing collection of an excessive amount of platelets operates, in the platelet collection operation except the last platelet collection operation, the stored data of the platelet collection condition, namely, the voltage of the collection termination turbidity sensor is dropped by a predetermined value to quicken the platelet collection operation. That is, the platelet concentration (for example, about 400,000/ml) in the normal platelet collection termination is altered to the platelet concentration (for example, about 900,000/ml) in the adjusted platelet collection termination. Thereby the time period for collecting the plasma containing high-concentration platelets becomes short. Thus the collection amount of platelets becomes smaller.

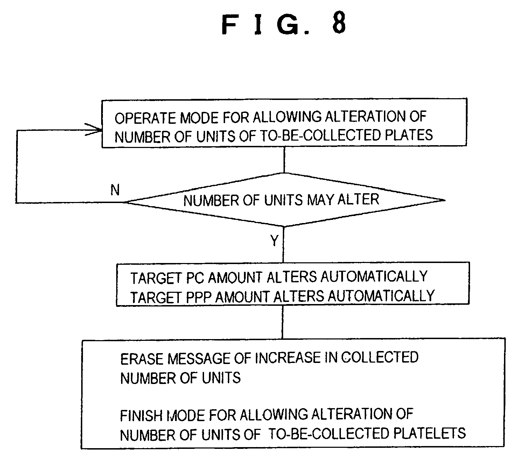

When it is determined that the number of collected platelets is on the "unit-increased level", a message indicating an increase in the number of units of collected platelets is displayed on the display part 52. Then the processing proceeds to a mode for allowing alteration of number of units of to-be-collected platelets shown in Fig. 8. In the mode for allowing alteration of number of units of to-be-collected platelets, it is possible to increase the target number of units of to-be-collected platelets. When the target number of units of to-be-collected platelets is increased, the target number of units of to-be-collected platelets is rewritten. Thereby the target collection amount of the plasma containing high-concentration platelets, the target collection amount of the platelets and plasma (PPP), and the collection condition are re-computed. Thereafter the message indicating an increase in the number of units of collected platelets is erased, and the execution of the mode for allowing alteration of number of units of to-be-collected platelets terminates. As described above, after whether the number of collected platelets is on the tendency to increase is determined, the buffy coat is collected, as shown in Fig. 7.

Upon completion of the platelet collection step, the huffy coat collection step is executed. In this step, the controller 13 doses the fourth flow path shutter means 84 and opens the fifth flow path shutter means 85. The plasma in the plasma collection bag 25 is fed to the centrifugal separator 20 by the blood pump 11. At the same time, the liquid that has been released (the huffy coat layer that has flowed out) from the centrifugal separator 20 flows into the buffy coat collection bag 27. In the huffy coat collection step, the final rate of the rotational speed of the blood pump in the platelet collection step is maintained, and the rotational speed of the centrifugal separator 20 is increased to the predetermined speed or a speed a little higher (higher by 50 - 200 rpm than the predetermined speed, for example, higher by 100 rpm) than the predetermined speed. In this manner, the huffy coat is released from the centrifugal separator 20 and collected in the buffy coat collection bag 27. At a time point when the amount of the collected buffy coat reaches a value computed based on the hematocrit value of the donor and the amount of collected platelet, the blood pump 11 is stopped and all the valves are closed. Thus the centrifugal separator 20 stops rotating, i.e., the execution of the buffy coat collection step terminates.

Then, the blood return step of returning the blood in the centrifugal separator 20 to the donor is executed. The controller 13 rotates the blood pump 11 in a reverse direction, opens the first flow path shutter means 81, and returns the red cell layer remaining in the centrifugal separator 20 to the donor through the first line 21.

Thus, the first (initial) platelet collection operation terminates.

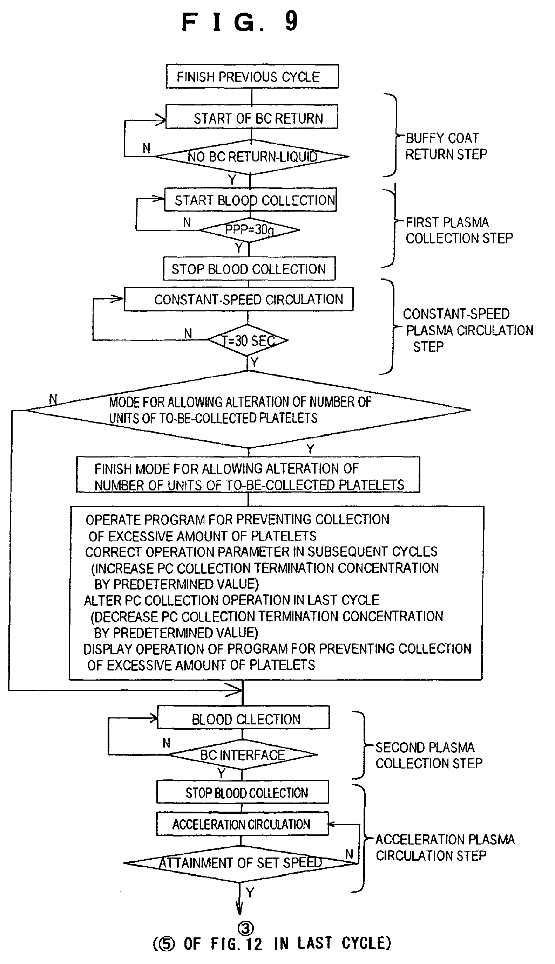

Then, the processing proceeds to a second platelet collection operation, as shown in Fig. 9.

Referring to Fig. 9, in the second cycle, initially the controller 13 executes the buffy coat return step of returning the buffy coat collected in the first platelet collection step to the centrifugal separator 20 before the next blood collection step is executed. Once the processing proceeds to the huffy coat return step, the controller 13 rotates the centrifugal separator 20 at a predetermined rotational speed (computed initial number of rotations of rotor), opens the fifth flow path shutter means 85 and the fourth flow path shutter means 84, and starts the blood pump 11 at a predetermined rate (default value: 100 ml/min). The huffy coat contained in the buffy coat collection bag 27 is fed to the centrifugal separator 20 through the fifth flow path shutter means 85. The air in the centrifugal separator 20 is sent to the platelet collection bag 26 through the second line 22 and the fourth flow path shutter means 84. After the blood pump 11 rotates in an amount corresponding to the set collection amount of the buffy coat, the execution of the buffy coat return step is completed.

Then, as shown in Fig. 9, the first plasma collection step and the constant-speed plasma circulation step are executed.

Whether the processing has proceeded to the mode for allowing alteration of number of units of to-be-collected platelets is determined. If YES, the execution of the determination processing terminates. Then the program for preventing collection of an excessive amount of platelets (function of decreasing the number of to-be-collected platelets) operates, which is displayed on the display part 52.

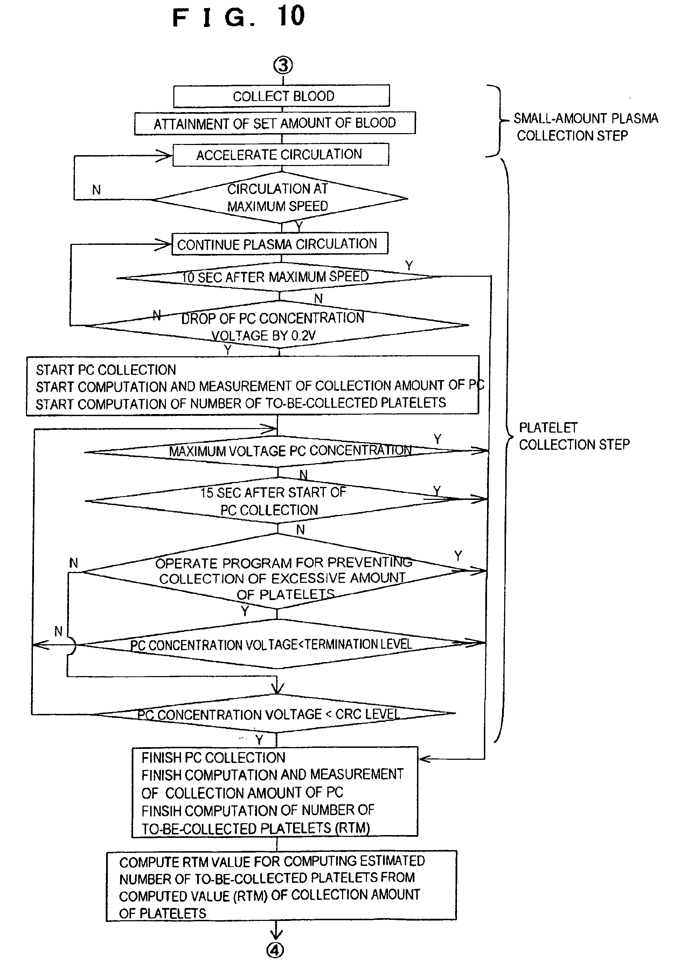

As shown in Fig. 9, after the second plasma collection step and the plasma circulation step are executed, the processing proceeds to the program referred to as ③ in Fig. 10 in which the small-amount plasma collection step and the platelet collection step are executed. In the platelet collection step, when the program for preventing collection of an excessive amount of platelets is in operation, the platelet collection operation terminates not when the platelet concentration reaches the CRC level (for example, about 400,000/ml in the normal platelet collection termination) but when the platelet concentration reaches about 900,000/ml (in the adjusted platelet collection termination). Thereby the time period for collecting the plasma containing high-concentration platelets becomes short. Thus the collection amount of platelets becomes smaller.

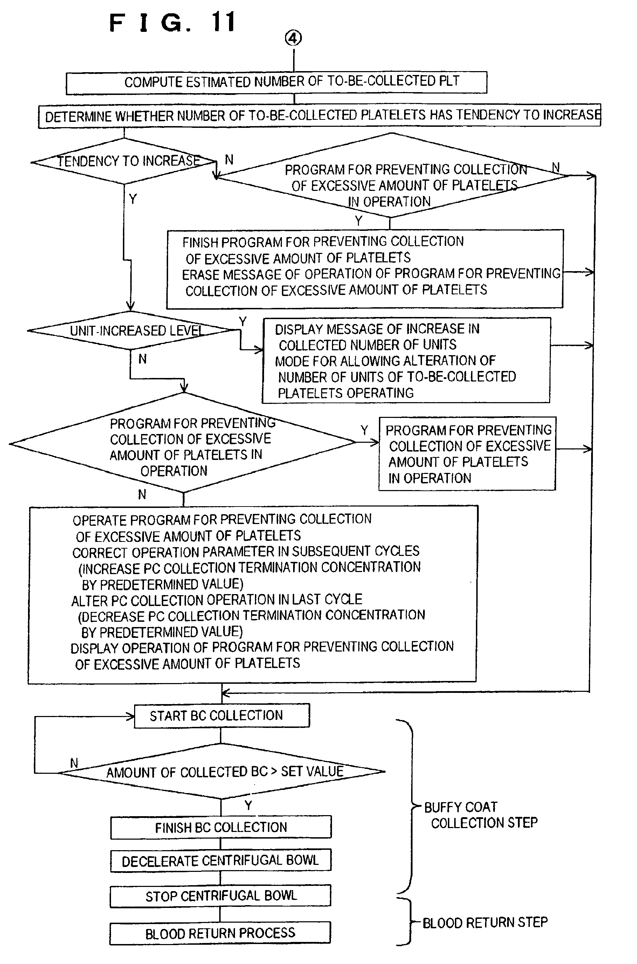

At the platelet collection step, regardless of whether the program for preventing collection of an excessive amount of platelets is in operation, similarly to the first cycle, computation and measurement of the collection amount of the plasma containing high-concentration platelets and computation of the number of collected platelets start, the number of collected platelets (RTM value) is computed, and the RTM-corrected value (RTM value for computation of estimated number of to-be-collected platelets) is computed. Then the processing proceeds to ④ of Fig. 11 in which it is determined whether the number of collected platelets has a tendency to increase.

If it is determined that there is "no tendency to increase (level of proper range)" in the number of collected platelets, the collection condition is not changed and the processing proceeds to the buffy coat collection step. If it is determined that the number of collected platelets has the "tendency to increase", it is determined whether the number of collected platelets is on the "unit-increased level". If it is determined that the number of collected platelets is not on the "unit-increased level", it is determined that the number of collected platelets is on the "level of tendency to increase". As a result, the program for preventing collection of an excessive amount of platelets (function of decreasing the number of to-be-collected platelets) operates, which is displayed on the display part 52. When it is determined that the number of collected platelets is on the "unit-increased level", the processing proceeds to the mode for allowing alteration of number of units of to-be-collected platelets. After whether the number of collected platelets is on the tendency to increase is determined, the buffy coat is collected, as shown in Fig. 11.

Then the buffy coat collection step and the blood return step are sequentially executed. In this manner, the second platelet collection operation terminates.

Description will be made on the last platelet collection operation with reference to Fig. 9. In this embodiment, the third operation is the last one. However, the fourth or subsequent platelet collection operations may be the last one. Each of these platelet collection operations except the last one is identical to the second platelet collection operation (Figs. 7 and 8).

In the last platelet collection operation, initially, as shown in Fig. 9, the buffy coat return step is executed. In this step, the buffy coat collected by the second platelet collection operation (previous platelet collection operation) is returned to the centrifugal separator 20 prior to the execution of the next plasma collection step.

When the processing proceeds to the buffy coat return step, the controller 13 rotates the centrifugal separator 20 at a predetermined rotational speed (for example, 4800 rpm), opens the fifth flow path shutter means 85 and the fourth flow path shutter means 84, and activates the blood pump 11 at a predetermined rate (default value: 100 ml/min). The buffy coat contained in the buffy coat collection bag 27 passes through the fifth flow path shutter means 85 and is supplied to the centrifugal separator 20. The air in the centrifugal separator 20 is sent to the platelet collection bag 26 through the second line 22 and the fourth flow path shutter means 84. After the blood pump 11 rotates in an amount corresponding to the set collection amount of the buffy coat, the execution of the buffy coat return step terminates.

Then, the first plasma collection step is executed. That is, the first and second liquid supply pumps 11, 12 are started to collect the anticoagulant-added blood, and the centrifugal separator drive unit 10 is activated to collect a first predetermined amount of plasma from the blood and flow it into the plasma collection bag 25.

Upon start of the first blood collection, the first liquid supply pump 11 is rotated at a predetermined rate (for example, 60 ml/min). At this time, the second pump constituting the anticoagulant pump supplies the anticoagulant (such as the ACD-A solution) at the predetermined rate (for example, 1/10 of the blood pump rate). Blood supplied from the donor is mixed with the ACD-A solution. The ACD-A solution-added blood flows through the first line 21, passes through the chamber and the first flow path shutter means 81, and flows into the centrifugal separator 20.

At this time, the sixth flow path shutter means 86, the fifth flow path shutter means 85, the second flow path shutter means 82, and the third flow path shutter means 83 are closed. Meanwhile the first flow path shutter means 81 and the fifth flow path shutter means 85 are opened. When the ACD-A solution-added blood is supplied to the centrifugal separator 20, the sterilized air that has been admitted into the centrifugal separator 20 flows into the buffy coat collection bag 27 through a line sensor and the fifth flow path shutter means 85. Simultaneously with the start of the blood collection step, the centrifugal separator 20 starts rotating at the predetermined speed. The centrifugal separator 20 is supplied with the ACD-A solution-added blood during its rotation. Thus, the centrifugal separator 20 separates it centrifugally into the plasma layer (inner layer), the buffy coat layer (BC layer, intermediate layer), and the red cell layer (outer layer). When about 270 ml of the ACD-A solution-added blood exceeding the capacity of the centrifugal separator is supplied to the centrifugal separator 20, it is filled with the blood, and the plasma overflows through the outlet of the centrifugal separator 20.

The turbidity sensor 14 mounted on the second line 22 connected to the outlet of the centrifugal separator 20 detects that the fluid flowing through the line has changed from the air to the plasma. Based on the detection signal outputted from the turbidity sensor 14, the controller 13 closes the fifth flow path shutter means 85 and opens the third flow path shutter means 83 to collect the plasma into the plasma collection bag 25. The weight of the plasma collection bag 25 is measured in advance by the weight sensor 16, and a signal indicating the measured weight is transmitted therefrom to the controller 13. Thus, when the weight of the plasma collected in the plasma collection bag 25 is increased to a predetermined amount (for example, 30 g), the controller 13 doses the first flow path shutter means 81 and opens the second flow path shutter means 82 to proceed to the constant-speed plasma circulation step.

Thereafter it is determined whether the processing has proceeded to the mode for allowing alteration of number of units of to-be-collected platelets. If YES, the determination processing terminates. Then the program for preventing collection of an excessive amount of platelets (function of decreasing the number of to-be-collected platelets) operates, which is displayed on the display part 52

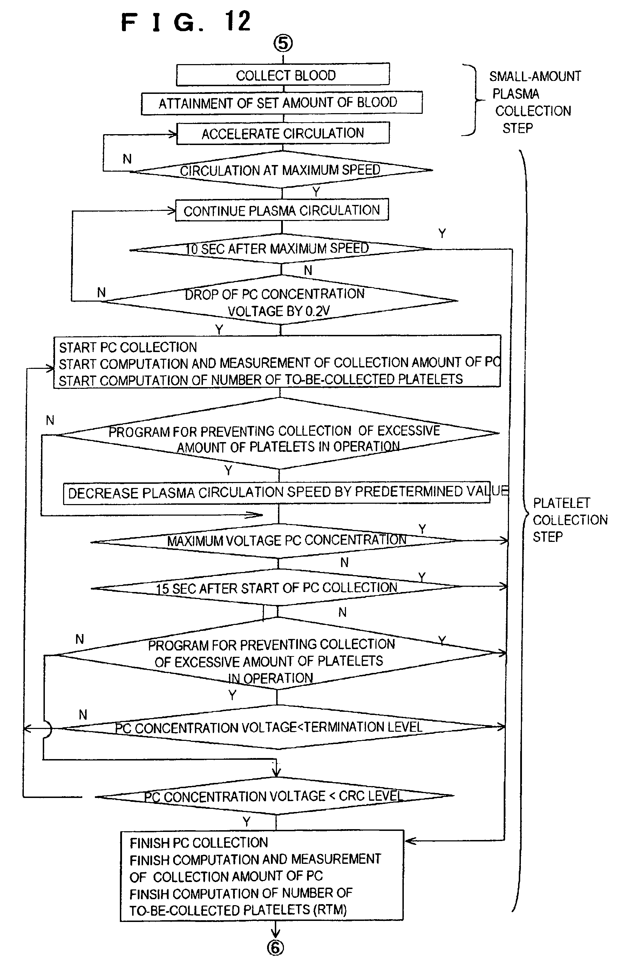

After the second plasma collection step and the acceleration plasma circulation step are executed, the processing proceeds to the program referred to as ⑤ in Fig. 12 in which a small-amount plasma collection step for interface adjustment and a platelet collection step are executed.

At the platelet collection step, when the program for preventing collection of an excessive amount of platelets is in operation after start of the collection of the plasma containing high-concentration platelets, the collection speed of the plasma containing high-concentration platelets is reduced to a predetermined level after platelets flow out. More specifically, the collection speed of the plasma containing high-concentration platelets is reduced from 200ml/min to 170ml/min. When the program for preventing collection of an excessive amount of platelets is in operation, the platelet concentration (for example, about 400,000/ml) in the normal platelet collection termination is altered to the platelet concentration (for example, about 300,000/ml) in the adjusted platelet collection termination. Thereby the degree of the concentration platelets in the plasma decreases a little, and the collection amount thereof is more than that collected in the normal condition.

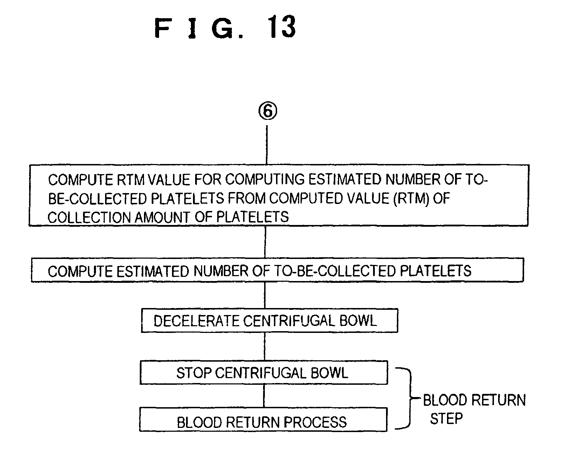

At the platelet collection step, regardless of whether the program for preventing collection of an excessive amount of platelets is in operation, similarly to the first cycle, computation and measurement of the collection amount of the plasma containing high-concentration platelets and computation of the number of collected platelets start, and the number of collected platelets (RTM value) is computed. Then the processing proceeds to ⑥ of Fig. 13 in which the RTM-corrected value (RTM value for computation of estimated number of to-be-collected platelets) is computed, and the estimated number of to-be-collected platelets is computed.

When the platelet collection step terminates, the blood return step is executed. Thereby the last platelet collection operation terminates.

The platelet collection apparatus has both the function of decreasing the number of to-be-collected platelets and the function of increasing the number of to-be-collected platelets. It is desirable that the platelet collection apparatus has both the functions. However, the platelet collection apparatus may have one of the two functions.

More specifically, the platelet collection apparatus may have only the function of decreasing the number of to-be-collected platelets. In this case, the platelet collection apparatus does not determine on "unit-increased level" in determining whether the number of collected platelets has a tendency to increase.

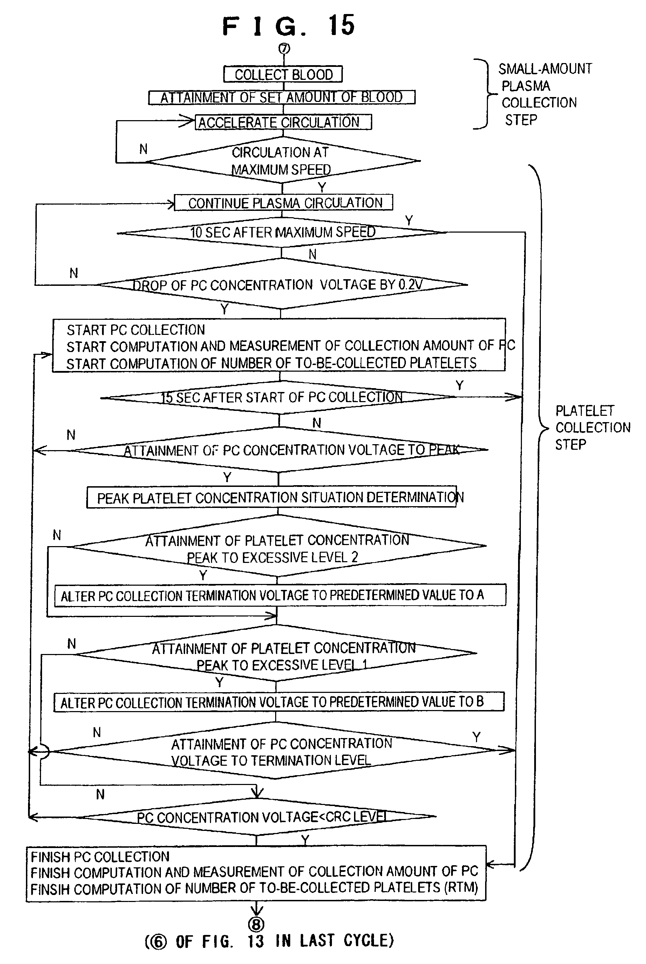

As shown in Fig. 15, even in the first platelet collection operation (first cycle), the platelet collection apparatus not having the function of increasing the number of to-be-collected platelets may have the function of decreasing the number of to-be-collected platelets by altering a set condition of the platelet collection operation. The platelet collection apparatus of this embodiment has the peak platelet concentration situation determination function of detecting the peak of the platelet concentration by using signals detected by the turbidity sensor when the high-concentration platelets-containing plasma is collected in the initial platelet collection operation and determining whether the detected peak of the platelet concentration is on the "normal level" or the "excessive level"; and the function of decreasing the number of platelets to be collected in the platelet collection operation, when the platelet-concentration peak situation determination function has determined that the peak of the platelet concentration is on the "excessive level".

The platelet collection apparatus of another embodiment is described below.

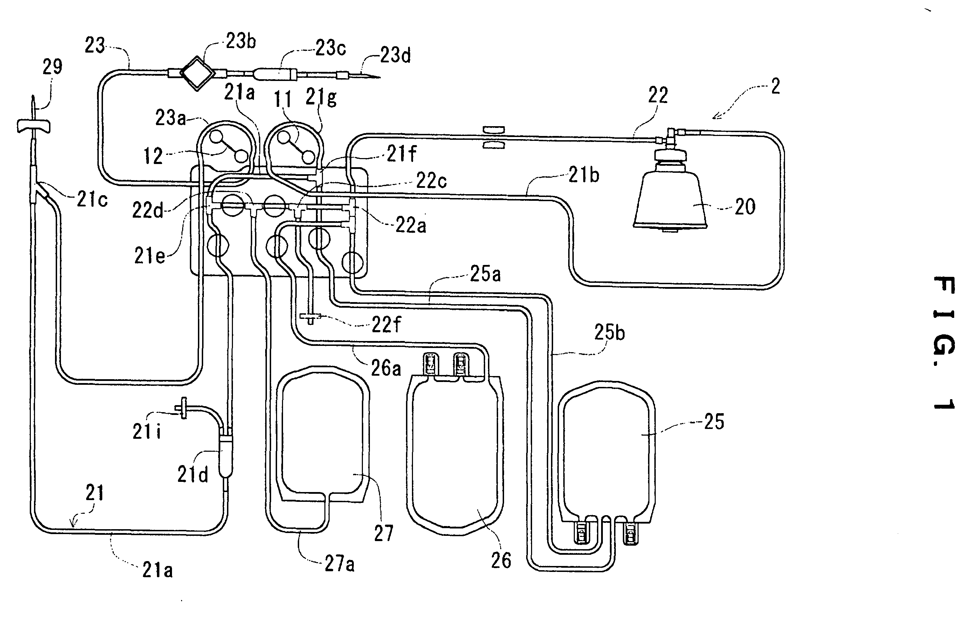

The platelet collection apparatus of this embodiment has a construction similar to that of the embodiment shown in Figs. 1 through 3.

The platelet collection apparatus of this embodiment has the peak platelet concentration situation determination function of detecting the peak of the platelet concentration by using signals detected by the turbidity sensor when the high-concentration platelets-containing plasma is collected in the platelet collection operation and determining whether the detected peak of the platelet concentration is on the "normal level" or the "excessive level"; and the function of decreasing the number of platelets to be collected in the platelet collection operation, when the platelet-concentration peak situation determination function has determined that the peak of the platelet concentration is on the "excessive level".

The platelet collection apparatus of this embodiment has only the function of decreasing the number of to-be-collected platelets.

The embodiment is described below with reference to Figs. 13 through 17.

The platelet collection apparatus of this embodiment has the function of decreasing the collection amount of platelets in the case where it is expected that the number of platelets to be collected in each platelet collection operation is more than a predetermined amount.

The peak platelet concentration situation determination function of this embodiment is similar to that of the above-described embodiment. The function of decreasing the collection amount of platelets of this embodiment is similar to that of the above-described embodiment.

It is preferable the platelet collection apparatus has an operation display function of informing an operator of the operation of the function of decreasing the collection amount of platelets. In addition to the automatic type, this function may be actuated by an operator's switching operation.

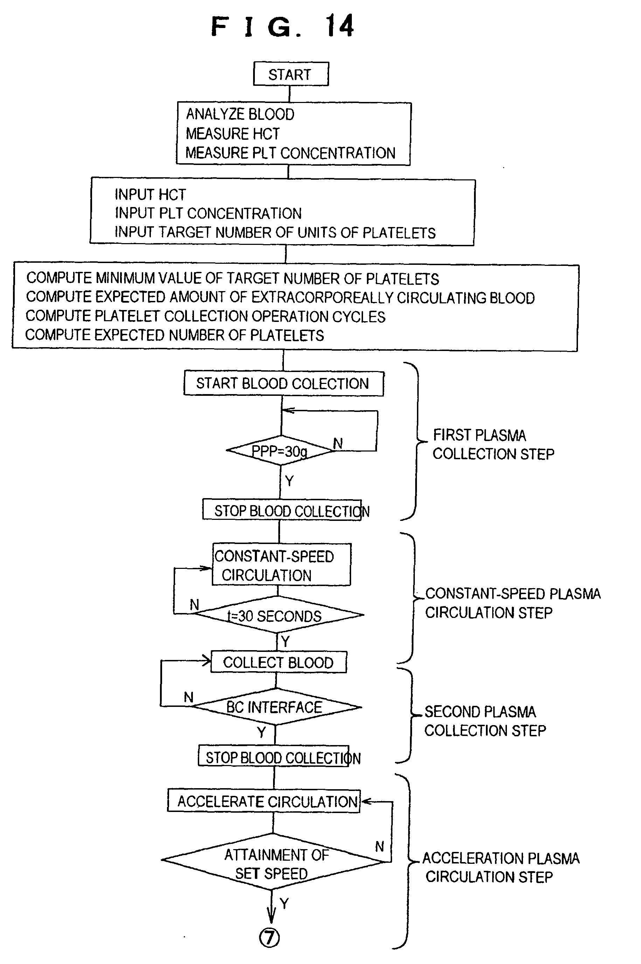

The platelet collection operation to be performed by the platelet collection apparatus of this embodiment is described in detail below with reference to Figs. 14 through 17.

As shown in Fig. 14, initially blood cell components are measured beforehand by using sampling blood collected from the donor immediately before an operator starts to collect the components of the blood. The hematcrit value (HCT) of the donor, a platelet concentration (PLT concentration) measured at this time, and the target number of units of to-be-collected platelets are inputted to the platelet collection apparatus. The control part of the platelet collection apparatus computes the minimum value of the target to-be-collected platelets, the amount of extracorporeally circulating blood, the number of platelet collection operation cycles, and the expected number of to-be-collected by using the above-described inputted values and data or computing equations stored therein. The control part may compute the expected number of units of to-be-collected platelets. Thereafter the number of platelet collection operation cycles and the expected number of to-be-collected platelets or the expected number of units of to-be-collected platelets are displayed on the display part 52. The control part 50 may have the function of computing an expected operation termination time, based on the number of platelet collection operation cycles and displaying results. The expected number of to-be-collected platelets that is computed by a method similar to that of the embodiment can be utilized.

First, the third line 23 and the blood collection needle 29 are primed with the anticoagulant. Then the blood collection needle 29 is stuck on the donor.

As shown in Figs. 14 and 15, between the first plasma collection step and the collection of the plasma containing high-concentration platelets (PC) at the platelet collection step, an operation similar to those of the above-described embodiments is performed (see Figs. 5 and 6). Thus description thereof is omitted herein.

Simultaneously with the start of the collection of the high-concentration platelets-containing plasma (PC), computation and measurement of the collection amount of the high-concentration platelets-containing plasma and computation of the number of collected platelets start. The voltage signals detected by the turbidity sensor are sequentially inputted to the control part. The amount of the plasma containing the high-concentration platelet is computed by using an operation amount of the liquid supply pump and a stored blood feeding amount per unit operation amount. The control part computes the number of collected platelets (RTM value) from the platelet concentration computed by using the voltage signal sent thereto from the turbidity sensor and a computed blood-feeding amount computed as described above and displays the number of collected platelets. The number of collected platelets (RTM value) can be obtained by computing the platelet concentration in a predetermined time period from output signals of the turbidity sensor detected at predetermined intervals (for example, 0.01 - 2 seconds) and multiplying the platelet concentration by the blood-feeding amount in the predetermined time period to thereby obtain the number of collected platelets in each predetermined time period. This operation is performed continuously in the time period in which the plasma containing the high-concentration platelet is collected. The total number of the collected platelets can be obtained by adding respective computed number of platelets to each other.

As shown in Fig. 15, when the collection of the plasma containing high-concentration platelets (PC) starts, whether the peak of the platelet concentration has been attained is determined, by using detection signals sent from the turbidity sensor. After the peak of the platelet concentration is attained, the situation of the peak of the platelet concentration is determined. If the peak of the platelet concentration corresponds to "excessive level 2", the platelet concentration (for example, about 400,000/ml) in the normal platelet collection termination is altered to a second platelet concentration (for example, about 900,000/ml) in the adjusted platelet collection termination. If the peak of the platelet concentration does not correspond to the "excessive level 2" but to an "excessive level 1", the platelet concentration (for example, about 400,000/ml) in the normal platelet collection termination is altered to a first platelet concentration (for example, about 900,000/ml) in the adjusted platelet collection termination. If the peak of the platelet concentration corresponds to neither the "excessive level 2" nor the "excessive level 1", the platelet concentration (for example, about 400,000/ml) in the normal platelet collection termination is maintained. When the peak of the platelet concentration reaches the platelet concentration in the normal platelet collection termination, the collection of the plasma containing high-concentration platelets terminates.

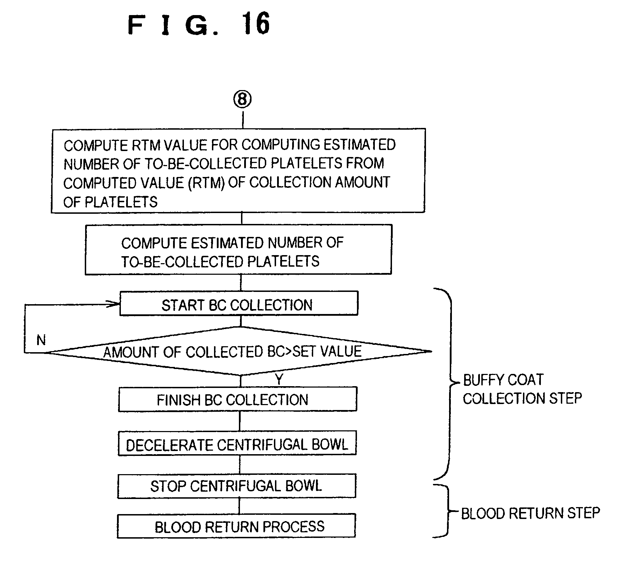

After the collection of the plasma containing high-concentration platelets terminates, the number of collected platelets (RTM value) is computed. Then the processing goes to ⑧ of Fig. 16 in which the RTM-corrected value (for computing estimated number of to-be-collected platelets) is computed. Then the display number of collected platelets is computed. Thereafter the buffy coat is collected The buffy coat collection step can be executed by a method similar to that of the above-described embodiments.

Then, the blood return step of returning the blood in the centrifugal separator 20 to the donor is executed. The blood return step can be executed by a method similar to that of the above-described embodiments. Thereby the first (initial) platelet collection operation terminates.

Then the processing proceeds to a second platelet collection operation.

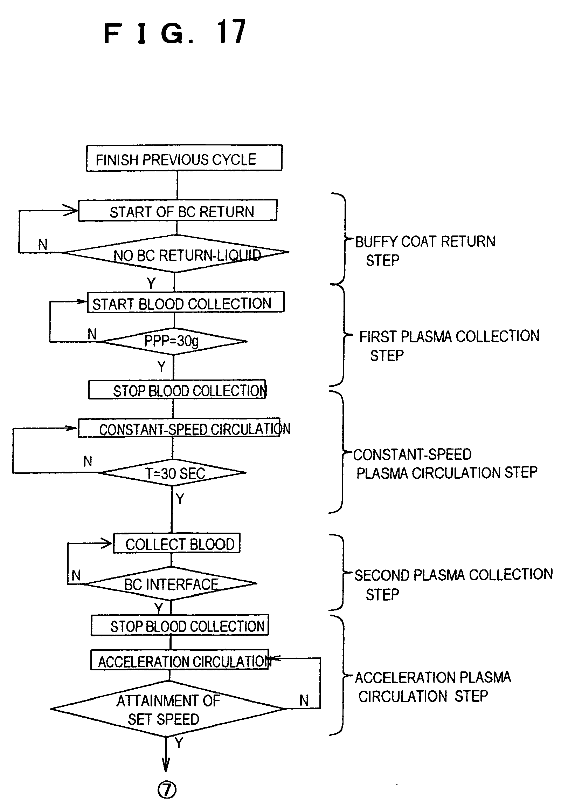

Referring to Fig. 17, in the second cycle, initially the controller 13 executes the buffy coat return step of returning the buffy coat collected in the first platelet collection step to the centrifugal separator 20 before the next blood collection step is executed. The buffy coat return step is executed by a method similar to that of the above-described embodiments.

Then, as shown in Fig. 17, the first plasma collection step, the constant-speed plasma circulation step, a second plasma collection step, and an acceleration plasma circulation step are executed. Then the processing proceeds to ⑦ of Fig. 15 in which the small-amount blood collection step and the platelet collection step are executed.

The platelet collection step is executed in a manner similar to that of the first cycle. That is, in a manner similar to that of the first cycle, when the collection of the plasma containing high-concentration platelets (PC) starts, computation and measurement of the collection amount of the plasma containing high-concentration platelets and computation of the number of collected platelets start. Computed number of collected platelets (RTM value: in second operation cycle and an n-th operation cycle, computed number of collected platelets in first operation cycle through (n-1)th operation cycle + RTM value in n-th operation cycle: total number of number of collected platelets) is displayed on the display part 52. While the plasma containing high-concentration platelets is being collected, it is determined whether the peak has been attained, by using signals detected by the turbidity sensor. When it is detected that the peak voltage has been attained, the peak platelet concentration situation is determined by using a detected result. As shown in Fig. 15 and as described previously, according to a result obtained by a determination made by peak platelet concentration situation determination function, the concentration of collected platelets is adjusted depending on necessity.

Then the RTM value for computing the estimated number of to-be-collected platelets is computed from the computed number of collected platelets (RTM). Thereafter the processing goes to ⑧ of Fig. 16 in which estimated number of to-be-collected platelets is computed from the computed RTM value for computing the estimated number of to-be-collected platelets. Then as shown in Fig. 16, the buffy coat step and the blood return step are sequentially executed. In this manner, the second platelet collection operation terminates.

Description will be made on the last platelet collection operation. In this embodiment, the third operation is the last one. However, the fourth or subsequent platelet collection operation may be the last one. Each of these platelet collection operations except the last one is identical to the second platelet collection operation (Figs. 7 and 8). The second platelet collection operation may be the last platelet collection operation.

In the last platelet collection operation, initially, as shown in Fig. 17, the huffy coat return step is executed. In this step, the buffy coat collected in the second platelet collection operation (previous platelet collection operation) is returned to the centrifugal separator 20 prior to the execution of the next plasma collection step. The buffy coat return step is executed by a method similar to that of the above-described embodiments.

Then, the first plasma collection step is executed. That is, the first and second liquid supply pumps 11, 12 are started to collect the anticoagulant-added blood, and the centrifugal separator drive unit 10 is activated to collect a first predetermined amount of plasma from the blood and flow it into the plasma collection bag 25. The first plasma collection step may be executed by a method similar to that of the above-described embodiments.

After the second plasma collection step and the acceleration plasma circulation step are executed, the processing proceeds to the program referred to as ⑦ in Fig. 15 in which the small-amount plasma collection step for interface adjustment and the platelet collection step are executed. The second plasma collection step, the acceleration plasma circulation step, and the small-amount plasma collection step for interface adjustment can be executed by a method similar to that of the first cycle or the above-described embodiments.

The platelet collection step is executed by a method similar to that of the first cycle. Upon termination of the platelet collection step, the blood return step is executed. Thereby the last platelet collection operation terminates.

The platelet collection apparatus of the present invention has a function of determining whether there is a tendency to increase or decrease in the number of collected platelets by using an estimated number of to-be-collected platelets computed by using an amount of plasma containing high-concentration platelets and a platelet concentration obtained from a turbidity sensor when the plasma is collected or data related to the platelet concentration and by using an expected number of to-be-collected platelets or a value related thereto; and a function of decreasing the amount of platelets to be collected in a subsequent platelet collection operation in the case where the function of determining on an increase/decrease tendency in the number of collected platelets has determined that there is a tendency to increase in the number of collected platelets.

Therefore it is possible to prevent platelets from being collected in an amount much larger than the target number of to-be-collected platelets.

The platelet collection apparatus of the present invention has a function of determining whether there is a tendency to increase or decrease in the number of collected platelets by using data, related to the concentration of platelets, obtained from a turbiditv sensor, the expected number of to-be-collected platelets or a value related thereto after the plasma (PC) containing high-concentration platelets is collected in a plasma collection operation; a function of altering an inputted target number of units of to-be-collected platelets in the case where the function of determining on an increase/decrease tendency in the number of collected platelets has determined that the number of units of collected platelets is on "unit-increased level"; and a function of increasing the amount of platelets to be collected in a subsequent platelet collection operation in the case where the function of altering the target number of units of to-be-collected platelets has altered the target number of units of to-be-collected platelets.

Therefore in the case where it is possible to increase the target number of units of platelets in dependence on a platelet-collected situation, it is possible to alter the target number of units of platelets and collect platelets at an altered target number of units thereof.

The platelet collection apparatus of the present invention has a function of determining whether there is a tendency to increase or decrease in the number of collected platelets by using data, related to the concentration of platelets, obtained from a turbidity sensor, an expected number of to-be-collected platelets or a value related thereto and determining on a "level of tendency to increase" and a "unit-increased level" which is higher than the "level of tendency to increase" after plasma (PC) containing high-concentration platelets is collected in a plasma collection operation; a function of altering an inputted target number of units of to-be-collected platelets in the case where the function of determining on an increase/decrease tendency in the number of collected platelets has determined that the number of units of platelets is on the "unit-increased level"; and a function of increasing an amount of platelets to be collected in a subsequent platelet collection operation in the case where the function of altering the target number of units of the to-be-collected platelets has altered the target number of units of the to-be-collected platelets; and a function of decreasing an amount of platelets to be collected in a subsequent platelet collection operation in the case where the function of altering the target number of units of the to-be-collected platelets has not altered the target number of units of platelets in a predetermined period of time after termination of an operation of the function of altering the target number of units of the to-be-collected platelets and in the case where the function of determining on an increase/decrease tendency in the number of collected platelets has determined that the number of the collected platelets is on the "level of tendency to increase".

Therefore in the case where it is possible to increase the target number of units of platelets in dependence on a platelet-collected situation, it is possible to alter the target number of units of platelets and collect platelets at an altered target number of units thereof. Further it is possible to prevent platelets from being collected in an amount much larger than the target number of to-be-collected platelets in the case where an increase is not desired in the target number of units of platelets or in the case where the target number of units of platelets is not on an increasable level although the target number of units of platelets has a tendency to increase.

The platelet collection apparatus of the present invention has a platelet-concentration peak situation determination function of detecting a peak of a platelet concentration by using signals detected by a turbidity sensor when the high-concentration platelets-containing plasma is collected in a platelet collection operation and determining whether a detected peak of the platelet concentration is on a "normal level" or an "excessive level"; and a function of decreasing the number of platelets to be collected in the platelet collection operation, when the platelet-concentration peak situation determination function has determined that the peak of the platelet concentration is on the "excessive level".

Therefore in the case where it is possible to increase the target number of units of platelets in dependence on a platelet-collected situation, it is possible to alter the target number of units of platelets and collect platelets at an altered target number of units thereof.

A platelet collection apparatus (1) has a function of determining whether there is a tendency to increase or decrease in the number of collected platelets by using data, related to the concentration of platelets, obtained from a turbidity sensor (14), an expected number of to-be-collected platelets or a value related thereto; and a function of decreasing the amount of platelets to be collected in a subsequent platelet collection operation after plasma containing high-concentration platelets is collected in a plasma collection operation, if the function of determining on an increase/ decrease tendency in the number of collected platelets determines that there is a tendency to increase in the number of collected platelets.

## Claims

1. A platelet collection apparatus (1) separating blood in to a plurality of components by means of a centrifugal separator (20) having a blood storage space therein and collecting at least platelets of said components, comprising:
a platelet collection circuit having a first line (21) connecting an inlet (143) of said centrifugal separator (20) and a blood collection means (29) with each other, a second line (22) connected with an outlet (144) of said centrifugal separator (20), and a platelet collection bag (26) connected with said second line (22);
a turbidity sensor (14) disposed on said second line (22); and
a function of computing a number of platelet collection cycles and an expected number of to-be-collected platelets or a value related thereto from data of a donor's hematcrit value, platelet concentration data, and a target number of said to-be-collected platelets, all of which are inputted to said platelet collection apparatus (1) before a platelet collection operation is performed,
a function of determining whether there is a tendency to increase or decrease in a number of collected platelets by using an estimated number of to-be-collected platelets computed by using an amount of plasma containing high-concentration platelets and a platelet concentration obtained from the turbidity sensor (14) when said plasma containing high-concentration platelets is collected or data related to said platelet concentration and by using said expected number of to-be-collected platelets or said value related thereto; and
a function of decreasing an amount of platelets to-be-collected in a subsequent platelet collection operation if said function of determining on an increase/decrease tendency in the number of collected platelets determines that there is a tendency to increase in the number of said collected platelets, said function of determining whether there is a tendency to increase or decrease in the number of collected platelets determines on a level of tendency to increase and a unit-increased level which is higher than said level of tendency to increase, if it is determined that there is a tendency to increase in the number of collected platelets, **characterized by**
a platelet concentration peak situation determination function of detecting a peak of a platelet concentration by using signals detected by said turbidity sensor (14), when high-concentration platelets-containing plasma is collected in a platelet collection operation and determining whether said detected peak of said platelet concentration is on a normal level or an excessive level;
a function of decreasing a number of platelets to-be-collected in said platelet collection operation, when said platelet-concentration peak situation determination function determines that said peak of said platelet concentration is on said excessive level, wherein
said platelet-concentration peak situation determination function determines whether a peak of a platelet concentration is on a normal level, a first excessive level or a second excessive level higher than said first excessive level, and
said function of decreasing the number of to-be-collected platelets alters data of a platelet collection termination condition, namely, a termination voltage of a turbidity sensor (14) to a first predetermined value, if said platelet concentration peak situation determination function determines that said peak of said platelet-concentration is on said first excessive level, and alters said termination voltage of said turbidity sensor (14) to a second predetermined value, if said platelet-concentration peak situation determination function determines that said peak of said platelet concentration is on said second excessive level.

2. A platelet collection apparatus (1) according to claim 1, further comprising a function of altering an inputted target number of units of to-be-collected platelets, if said function of determining on an increase/decrease tendency in the number of collected platelets determines that the number of units of platelets is on the unit-increased level.

3. A platelet collection apparatus (1) according to claim 2, further comprising a function of increasing an amount of platelets to-be-collected in a subsequent platelet collection operation, if said function of altering the target number of units of to-be-collected platelets has altered said target number of units of said to-be-collected platelets, and
a function of decreasing an amount of platelets to-be-collected in a subsequent platelet collection operation, if said function of altering said target number of units of the to-be-collected platelets has not altered the target number of units of platelets in a predetermined period of time after termination of an operation of said function of altering the target number of units of the to-be-collected platelets and if said function of determining on an increase/decrease tendency in the number of collected platelets determines that the number of the collected platelets is on said level of tendency to increase.

4. A platelet collection apparatus (1) according to claim 2 or 3, wherein when said function of altering an inputted target number of units of to-be-collected platelets alters a target number of units of to-be-collected platelets, a computing portion of a control part re-computes an expected number of to-be-collected platelets or a value related thereto, and a function of determining on an increase tendency in the number of collected platelets makes a determination by using a re-computed expected number of to-be-collected platelets or a value related thereto.

5. A platelet collection apparatus (1) according to any one of the preceding claims, wherein said function of decreasing the number of to-be-collected platelets alters stored data of a platelet collection termination condition to quicken a platelet collection operation and decrease a collection amount of said plasma containing high concentration platelets.

6. A platelet collection apparatus (1) according to any one of the preceding claims, wherein said platelet collection operation includes a plasma collection/circulation operation, a high-concentration platelets-containing plasma collection operation after the plasma collection/circulation operation and a blood return operation after the plasma containing high-concentration platelets collection operation.

7. A platelet collection apparatus (1) according to any one of the preceding claims, further comprising:
an expected collection-amount-group determination function of determining which of stored expected collection-amount-groups corresponds to the number of collected platelets, by using the expected number of to-be-collected platelets or the value related thereto, and
a function of computing the estimated number of to-be-collected platelets, by using data related to the collection amount of the high-concentration platelets-containing plasma in the platelet collection operation and data related to the platelet concentration obtained from the turbidity sensor when the high-concentration platelets-containing plasma is collected, and
said function of determining an increase/decrease tendency in the number of collected platelets determines an increase tendency by using the data of the corresponding collection-amount-group determined by the collection-amount-group determination function and the estimated number of to-be-collected platelets computed by the function of computing the estimated number of to-be-collected platelets.

8. A platelet collection apparatus (1) according to any one of the preceding claims, wherein said platelet collection apparatus performs the platelet collection operation at least three times, and in the platelet collection operation except the last platelet collection operation, the function of decreasing the number of to-be-collected platelets alters the stored data of the platelet collection termination condition to quicken the platelet collection operation and decrease the collection amount of the plasma containing high-concentration platelets, and in the last platelet collection operation, the function of decreasing the number of to-be-collected platelets alters the stored data of the platelet collection condition to decrease the concentration of platelets contained in the plasma containing the high-concentration platelets.

## Patentansprüche

1. Plättchensammelvorrichtung (1), die Blut mittels einer zentrifugalen Trenneinrichtung (20) mit einem Blutspeicherraum darin in eine Vielzahl von Komponenten trennt und zumindest Plättchen der Komponenten sammelt,
die folgendes aufweist:
einen Plättchensammelkreis mit einer ersten Leitung (21), die einen Einlass (143) der zentrifugalen Trenneinrichtung (20) und eine Blutsammeleinrichtung (29) miteinander verbindet, einer zweiten Leitung (22), die mit einem Auslass (144) der zentrifugalen Trenneinrichtung (20) verbunden ist, und einem Plättchensammelbeutel (26), der mit der zweiten Leitung (22) verbunden ist;
einen Trübungssensor (14), der an der zweiten Leitung (22) angeordnet ist; und
eine Funktion eines Berechnens einer Anzahl von Plättchensammelzyklen und einer erwarteten Anzahl von zu sammelnden Plättchen oder einem Wert, der sich darauf bezieht, aus Daten eines Spenderhämatokritwerts, Plättchenkonzentrationsdaten und einer Sollanzahl von zu sammelnden Plättchen, von denen alle an die Plättchensammelvorrichtung (1) eingegeben werden, bevor ein Plättchensammelbetrieb durchgeführt ist,
eine Funktion eines Bestimmens, ob eine Tendenz einer Zunahme oder Abnahme in einer Anzahl von gesammelten Plättchen vorhanden ist unter Verwendung einer geschätzten Anzahl von zu sammelnden Plättchen, die durch ein Verwenden einer Menge von Plasma, die eine hohe Konzentration Plättchen enthält, und einer Plättchenkonzentration, die von dem Trübungssensor (14) erhalten ist, wenn das Plasma, das eine hohe Konzentration an Plättchen enthält, gesammelt ist ,oder aus Daten berechnet ist, die sich auf die Plättchenkonzentration beziehen, und unter Verwendung der erwarteten Anzahl von zu sammelnden Plättchen oder dem zugehörigen Wert; und
eine Funktion eines Verringerns einer Menge von Plättchen, die in einem nachfolgenden Plättchensammelbetrieb zu sammeln sind, falls die Funktion eines Bestimmens einer Zunahme-/Abnahmetendenz in der Anzahl von gesammelten Plättchen bestimmt, dass es eine Tendenz einer Zunahme in der Anzahl der gesammelten Plättchen gibt, wobei die Funktion eines Bestimmens, ob eine Tendenz einer Zunahme oder Abnahme in der Anzahl von gesammelten Plättchen besteht, auf einem Niveau einer Tendenz einer Zunahme und einem einheitserhöhten Niveau, das höher als das Niveau einer Tendenz einer Zunahme ist, bestimmt, ob bestimmt ist, dass es eine Tendenz einer Zunahme in der Anzahl der gesammelten Plättchen gibt,
**gekennzeichnet durch**
eine Bestimmungsfunktion einer Plättchenkonzentrationsspitzensituation eines Erfassens einer Spitze einer Plättchenkonzentration **durch** Verwenden von Signalen, die **durch** den Trübungssensor (14) erfasst sind, wenn ein hochkonzentriertes Plättchen enthaltendes Plasma in einem Plättchensammelbetrieb gesammelt ist, und eines Bestimmens, ob die erfasste Spitze der Plättchenkonzentration auf einem normalen Niveau oder einem übermäßigen Niveau ist;
eine Funktion eines Verringerns einer Anzahl von zu sammelnden Plättchen in dem Plättchensammelbetrieb, wenn die Bestimmungsfunktion einer Plättchenkonzentrationsspitzensituation bestimmt, dass die Spitze der Plättchenkonzentration auf dem übermäßigen Niveau ist, wobei
die Bestimmungsfunktion einer Plättchenkonzentrationsspitzensituation bestimmt, ob eine Spitze einer Plättchenkonzentration auf einem normalen Niveau, einem ersten übermäßigen Niveau oder einem zweiten übermäßigen Niveau ist, das höher als das erste übermäßige Niveau ist, und
die Funktion eines Verringerns der Anzahl von zu sammelnden Plättchen Daten einer Plättchensammelbeendigungsbedingung ändert, nämlich eine Beendigungsspannung eines Trübungssensors (14) auf einen ersten vorbestimmten Wert, falls die Bestimmungsfunktion einer Plättchenkonzentrationsspitzensituation bestimmt, dass die Spitze der Plättchenkonzentration an dem ersten übermäßigen Niveau ist, und die Beendigungsspannung des Trübungssensors (14) auf einen zweiten vorbestimmten Wert ändert, falls die Bestimmungsfunktion einer Plättchenkonzentrationsspitzensituation bestimmt, dass die Spitze der Plättchenkonzentration an dem zweiten übermäßigen Niveau ist.

2. Plättchensammelvorrichtung (1) nach Anspruch 1, ferner mit einer Funktion eines Änderns einer eingegebenen Sollanzahl von Einheiten von zu sammelnden Plättchen, falls die Funktion eines Bestimmens einer Zunahme-/Abnahmetendenz in der Anzahl von gesammelten Plättchen bestimmt, dass die Anzahl von Einheiten von Plättchen auf dem einheitserhöhten Niveau ist.

3. Plättchensammelvorrichtung (1) nach Anspruch 2, ferner mit einer Funktion eines Erhöhens einer Anzahl von zu sammelnden Plättchen in einem nachfolgenden Plättchensammelbetrieb, falls die Funktion des Änderns der Sollanzahl von Einheiten von zu sammelnden Plättchen die Sollanzahl von Einheiten der zu sammelnden Plättchen verändert hat, und
einer Funktion eines Verringerns einer Anzahl von zu sammelnden Plättchen in einem nachfolgenden Plättchensammelbetrieb, falls die Funktion des Änderns der Sollanzahl von Einheiten der zu sammelnden Plättchen die Sollanzahl von Einheiten von Plättchen in einer vorbestimmten Zeitdauer nach einer Beendigung eines Betriebs der Funktion des Änderns der Sollanzahl von Einheiten der zu sammelnden Plättchen nicht geändert hat und falls die Funktion des Bestimmens einer Zunahme-/Abnahmetendenz in der Anzahl von gesammelten Plättchen bestimmt, dass die Anzahl der gesammelten Plättchen auf dem Niveau einer Zunahmetendenz ist.

4. Plättchensammelvorrichtung (1) nach Anspruch 2 oder 3, wobei dann, wenn die Funktion des Änderns einer eingegebenen Sollanzahl von Einheiten von zu sammelnden Plättchen eine Sollanzahl von Einheiten von zu sammelnden Plättchen ändert, ein Berechnungsabschnitt eines Steuerteils eine erwartete Anzahl von zu sammelnden Plättchen oder einen zugehörigen Wert neu berechnet und eine Funktion des Bestimmens einer Zunahmetendenz in der Anzahl von gesammelten Plättchen eine Bestimmung vornimmt unter Verwendung einer erneut berechneten, erwarteten Anzahl von zu sammelnden Plättchen oder einem zugehörigen Wert.

5. Plättchensammelvorrichtung (1) nach einem der vorangehenden Ansprüche, wobei die Funktion des Verringerns der Anzahl von zu sammelnden Plättchen gespeicherte Daten einer Plättchensammelbeendigungsbedingung ändert, um einen Plättchensammelbetrieb zu beschleunigen und um eine Sammelmenge des Plasmas, das hochkonzentrierte Plättchen enthält, zu verringern.

6. Plättchensammelvorrichtung (1) nach einem der vorangehenden Ansprüche, wobei der Plättchensammelbetrieb einen Plasmasammel-/- Zirkulationsbetrieb, einen Sammelbetrieb von hochkonzentrierten Plättchen enthaltenden Plasma nach dem Plasmasammel-/- Zirkulationsbetrieb und einen Blutrückgabebetrieb nach dem Sammelbetrieb des Plasmas, das hochkonzentrierte Plättchen enthält, aufweist.

7. Plättchensammelvorrichtung (1) nach einem der vorangehenden Ansprüche, die ferner folgendes aufweist:
eine Erwartungssammelmengengruppenbestimmungsfunktion eines Bestimmens, welche der gespeicherten erwarteten Sammelmengengruppen der Anzahl von gesammelten Plättchen entspricht, unter Verwendung der erwarteten Anzahl von zu sammelnden Plättchen oder dem zugehörigen Wert, und
eine Funktion eines Berechnens der erwarteten Anzahl von zu sammelnden Plättchen, unter Verwendung von Daten, die sich auf die Sammelmenge des hochkonzentrierte Plättchen enthaltenden Plasmas in dem Plättchensammelbetrieb beziehen, und Daten, die sich auf die Plättchenkonzentration beziehen, die von dem Trübungssensor erhalten ist, wenn das hochkonzentrierte Plättchen enthaltende Plasma gesammelt ist, und
die Funktion des Bestimmens einer Zunahme-/Abnahmetendenz in der Anzahl von gesammelten Plättchen eine Zunahmetendenz unter Verwendung der Daten der entsprechenden Sammelmengengruppe, die durch die Sammelmengengruppenbestimmungsfunktion bestimmt ist, und die geschätzte Anzahl von zu sammelnden Plättchen bestimmt, die durch die Funktion des Berechnens der geschätzten Anzahl von zu sammelnden Plättchen berechnet ist.

8. Plättchensammelvorrichtung (1) nach einem der vorangehenden Ansprüche, wobei die Plättchensammelvorrichtung den Plättchensammelbetrieb wenigstens dreimal durchführt und in dem Plättchensammelbetrieb außer in dem letzten Plättchensammelbetrieb die Funktion des Verringerns der Anzahl von zu sammelnden Plättchen die gespeicherten Daten der Plättchensammelbeendigungsbedingung ändert, um den Plättchensammelbetrieb zu beschleunigen und um die Sammelmenge des Plasmas, das hochkonzentrierte Plättchen enthält, zu verringern, und in dem letzten Plättchensammelbetrieb die Funktion des Verringerns der Anzahl von zu sammelnden Plättchen die gespeicherten Daten der Plättchensammelbedingung ändert, um die Konzentration der Plättchen zu verringern, die in dem Plasma enthalten ist, das die hochkonzentrierten Plättchen enthält.

## Revendications

1. Appareil de collecte de plaquettes (1) séparant le sang en une pluralité de composants à l'aide d'un séparateur centrifuge (20) ayant à l'intérieur un espace de stockage de sang et collectant au moins des plaquettes desdits composants, comprenant :
un circuit de collecte de plaquettes ayant une première ligne (21) reliant entre eux une admission (143) dudit séparateur centrifuge (20) et un moyen de collecte de sang (29), une deuxième ligne (22) reliée à une sortie (144) dudit séparateur centrifuge (20), et un sac de collecte de plaquettes (26) relié à ladite deuxième ligne (22) ;
un capteur de turbidité (14) disposé sur ladite deuxième ligne (22) ; et
une fonction de calcul d'un nombre de cycles de collecte de plaquettes et d'un nombre prévu de plaquettes à collecter ou d'une valeur qui lui est liée d'après des données de la valeur de l'hématocrite d'un donneur, des données de concentration des plaquettes, et d'un nombre cible desdites plaquettes à collecter, qui sont tous introduits dans ledit appareil de collecte de plaquettes (1) avant de réaliser une opération de collecte de plaquettes,
une fonction destinée à déterminer s'il y a une tendance à l'augmentation ou à la diminution d'un nombre de plaquettes collectées en utilisant un nombre estimatif de plaquettes à collecter calculé en utilisant une quantité de plasma contenant des plaquettes à haute concentration et une concentration de plaquettes obtenue du capteur de turbidité (14) lorsque ledit plasma contenant des plaquettes à haute concentration est collecté ou des données liées à ladite concentration de plaquettes et en utilisant ledit nombre prévu de plaquettes à collecter ou ladite valeur qui lui est liée ; et
une fonction destinée à diminuer une quantité de plaquettes à collecter dans une opération ultérieure de collecte de plaquettes si ladite fonction destinée à déterminer une tendance à l'augmentation/à la diminution du nombre de plaquettes collectées détermine qu'il y a une tendance à l'augmentation du nombre desdites plaquettes collectées, ladite fonction destinée à déterminer s'il y a une tendance à l'augmentation ou la diminution du nombre de plaquettes collectées détermine selon un niveau d'une tendance à l'augmentation et un niveau d'augmentation des unités qui est supérieur audit niveau de la tendance à l'augmentation, si l'on détermine qu'il y a une tendance à l'augmentation du nombre de plaquettes collectées, **caractérisé par**
une fonction de détermination de la situation du pic de concentration de plaquettes pour détecter un pic d'une concentration de plaquettes en utilisant des signaux détectés par ledit capteur de turbidité (14), lorsque ledit plasma contenant des plaquettes à haute concentration est collecté dans une opération de collecte de plaquettes et pour déterminer si ledit pic détecté de ladite concentration de plaquettes se trouve à un niveau normal ou à un niveau élevé ;
une fonction de diminution du nombre de plaquettes à collecter dans ladite opération de collecte de plaquettes, lorsque ladite fonction de détermination de la situation du pic de concentration de plaquettes détermine que ledit pic de ladite concentration de plaquettes est audit niveau élevé, dans lequel
ladite fonction de détermination de la situation du pic de concentration de plaquettes détermine si un pic d'une concentration de plaquettes est à un niveau normal, à un premier niveau élevé ou à un deuxième niveau élevé supérieur audit premier niveau élevé, et
ladite fonction de diminution du nombre de plaquettes à collecter modifie les données d'un état de fin de la collecte de plaquettes, soit, une tension de fin d'un capteur de turbidité (14) à une première valeur prédéterminée, si ladite fonction de détermination de la situation du pic de la concentration de plaquettes détermine que ledit pic de ladite concentration de plaquettes est audit premier niveau élevé, et modifie ladite tension de fin dudit capteur de turbidité (14) à une deuxième valeur prédéterminée, si ladite fonction de détermination de la situation du pic de la concentration de plaquettes détermine que ledit pic de ladite concentration de plaquettes est audit deuxième niveau élevé.

2. Appareil de collecte de plaquettes (1) selon la revendication 1, comprenant en outre une fonction de modification d'un nombre cible introduit d'unités de plaquettes à collecter, si ladite fonction de détermination d'une tendance à l'augmentation/à la diminution du nombre de plaquettes collectées détermine que le nombre d'unités de plaquettes est au niveau d'augmentation des unités.

3. Appareil de collecte de plaquettes (1) selon la revendication 2, comprenant en outre une fonction destinée à augmenter la quantité de plaquettes à collecter dans une opération ultérieure de collecte de plaquettes, si ladite fonction de modification du nombre cible d'unités des plaquettes à collecter a modifié ledit nombre cible d'unités desdites plaquettes à collecter, et
une fonction de diminution d'une quantité de plaquettes à collecter dans une opération ultérieure de collecte de plaquettes, si ladite fonction de modification dudit nombre cible d'unités de plaquettes à collecter n'a pas modifié le nombre cible d'unités de plaquettes dans un période prédéterminée après la fin d'une opération de ladite fonction de modification du nombre cible d'unités de plaquettes à collecter et si ladite fonction de détermination d'une tendance à l'augmentation/à la diminution du nombre de plaquettes collectées détermine que le nombre des plaquettes collectées est audit niveau d'une tendance à l'augmentation.

4. Appareil de collecte de plaquettes (1) selon la revendication 2 ou 3, dans lequel, lorsque ladite fonction de modification d'un nombre cible introduit d'unités de plaquettes à collecter modifie un nombre cible d'unités de plaquettes à collecter, une partie de calcul d'une partie de commande recalcule un nombre prévu de plaquettes à collecter ou une valeur qui lui est liée, et une fonction de détermination d'une tendance à l'augmentation du nombre de plaquettes collectées fait une détermination en utilisant un nombre prévu recalculé des plaquettes à collecter ou une valeur qui lui est liée.

5. Appareil de collecte de plaquettes (1) selon l'une quelconque des revendications précédentes, dans lequel ladite fonction destiné à diminuer le nombre de plaquettes à collecter modifie les données stockées d'un état de fin de la collecte de plaquettes afin d'accélérer une opération de collecte de plaquettes et de diminuer la quantité de collecte dudit plasma contenant des plaquettes à haute concentration.

6. Appareil de collecte de plaquettes (1) selon l'une quelconque des revendications précédentes, dans lequel ladite opération de collecte de plaquettes comporte une opération de collecte/de circulation de plasma, une opération de collecte de plasma contenant des plaquettes à haute concentration après l'opération de collecte/de circulation de plasma et une opération de retour de sang après l'opération de collecte du plasma contenant des plaquettes à haute concentration.

7. Appareil de collecte de plaquettes (1) selon l'une quelconque des revendications précédentes, comprenant en outre :
une fonction de détermination de groupes de quantités de collecte prévus pour déterminer lequel parmi des groupes de quantités de collecte prévus stockés correspond au nombre de plaquettes collectées, en utilisant le nombre prévu de plaquettes à collecter ou la valeur qui lui est liée, et
une fonction de calcul du nombre estimatif des plaquettes à collecter, en utilisant des données liées à la quantité de collecte de plasma contenant des plaquettes à haute concentration dans l'opération de collecte de plaquettes et des données liées à la concentration de plaquettes obtenues par le capteur de turbidité lorsque le plasma contenant des plaquettes à haute concentration est collecté, et
ladite fonction destinée à déterminer une tendance à l'augmentation/à la diminution du nombre de plaquettes collectées détermine une tendance à l'augmentation en utilisant les données du groupe de quantités de collecte correspondant déterminé par la fonction de détermination de groupes de quantités de collecte et le nombre estimatif des plaquettes à collecter calculé par la fonction de calcul du nombre estimatif des plaquettes à collecter.

8. Appareil de collecte de plaquettes (1) selon l'une quelconque des revendications précédentes, dans lequel ledit appareil de collecte de plaquettes réalise l'opération de collecte de plaquettes au moins trois fois, et dans l'opération de collecte de plaquettes, à l'exception de la dernière opération de collecte de plaquettes, la fonction de diminution du nombre de plaquettes à collecter modifie les données stockées de l'état de fin de collecte des plaquettes afin d'accélérer l'opération de collecte de plaquettes et de diminuer la quantité de collecte du plasma contenant des plaquettes à haute concentration, et dans la dernière opération de collecte de plaquettes, la fonction destinée à diminuer le nombre de plaquettes à collecter modifie les données stockées de l'état de collecte de plaquettes afin de diminuer la concentration de plaquettes contenues dans le plasma contenant les plaquettes à haute concentration.
